# EUROPEAN PATENT SPECIFICATION

(11) **EP 3 684 936 B1**
(45) Date of publication and mention of the grant of the patent: **06.12.2023**
(21) Application number: 18782634.2
(22) Date of filing: 12.09.2018
(51) Int. Cl.: C12N 15/82, C07K 14/47

(54) **METHOD OF PRODUCTION OF BARLEY PLANTS PRODUCING ANTIMICROBIAL PEPTIDES**
VERFAHREN ZUR HERSTELLUNG VON ANTIMIKROBIELLE PEPTIDE PRODUZIERENDEN GERSTENPFLANZEN
PROCÉDÉ DE PRODUCTION DE PLANTES D'ORGE PRODUISANT DES PEPTIDES ANTIMICROBIENS

(30) Priority: 18.09.2017 CZ 20170553
(43) Date of publication of application: 29.07.2020
(73) Proprietor: Usovsko A.S., 78973 Klopina, Usov (CZ); Univerzita Palackého v Olomouci, 779 00 Olomouc (CZ)
(72) Inventor: HOLASKOVA, Edita, 77900 Olomouc (CZ); FREBORT, Ivo, 77900 Bystrocice (CZ); MILEK, Jiri, 78973 Klopina Usov (CZ)
(74) Representative: Hartvichova, Katerina
(86) International application number: PCT/CZ2018/050048
(87) International publication number: WO 2019/052588

(56) References cited:
- WO-A2-2007/081487
- WO-A2-2008/140582
- EDITA HOLÁSKOVÁ ET AL: "Molecular Farming in Barley: Development of a Novel Production Platform to Produce Human Antimicrobial Peptide LL-37", BIOTECHNOLOGY JOURNAL, 8 February 2018 (2018-02-08), page 1700628, XP055524766, DE ISSN: 1860-6768, DOI: 10.1002/biot.201700628
- KATARÍNA MRÍZOVÁ ET AL: "Transgenic barley: A prospective tool for biotechnology and agriculture", BIOTECHNOLOGY ADVANCES., vol. 32, no. 1, 1 January 2014 (2014-01-01), pages 137-157, XP055524794, GB ISSN: 0734-9750, DOI: 10.1016/j.biotechadv.2013.09.011
- LÝ UR S. ERLENDSSON ET AL: "Barley as a green factory for the production of functional Flt3 ligand", BIOTECHNOLOGY JOURNAL, vol. 5, no. 2, 20 October 2009 (2009-10-20), pages 163-171, XP055524856, DE ISSN: 1860-6768, DOI: 10.1002/biot.200900111
- IN HYE LEE ET AL: "SP-LL-37, human antimicrobial peptide, enhances disease resistance in transgenic rice", PLOS ONE, vol. 12, no. 3, 10 March 2017 (2017-03-10) , page e0172936, XP055525454, DOI: 10.1371/journal.pone.0172936
- GOETZ HENSEL ET AL: "Transgenic Production of an Anti HIV Antibody in the Barley Endosperm", PLOS ONE, vol. 10, no. 10, 13 October 2015 (2015-10-13), page e0140476, XP055375088, DOI: 10.1371/journal.pone.0140476
- YU-JIN JUNG ET AL: "Enhanced resistance to bacterial and fungal pathogens by overexpression of a human cathelicidin antimicrobial peptide (hCAP18/LL-37) in Chinese cabbage", PLANT BIOTECHNOLOGY REPORTS, SPRINGER JAPAN, JAPAN, vol. 6, no. 1, 11 October 2011 (2011-10-11), pages 39-46, XP035004774, ISSN: 1863-5474, DOI: 10.1007/S11816-011-0193-0

## Description

### Field of Art

The present invention relates to a process for the production of genetically modified barley plants that produce recombinant biologically active antimicrobial peptides, in particular antimicrobial peptides from the cathelicidin group, suitable for molecular farming. Further, a method for their easy purification from barley grains is described. Recombinant products may be used after isolation and purification in pharmaceutical, cosmetic, veterinary or plant protection industries.

### Background Art

Antimicrobial peptides (AMPs) are effector molecules that are an essential part of the humoral component of the innate immune system of almost all living organisms. AMPs can be categorized based on structural motifs into 4 basic groups, namely beta-sheet peptides, alpha-helical peptides, peptides with loose structure and hairpin structures. Most AMPs are formed by less than 50 amino acid residues and share several common physicochemical properties, such as total positive charge (significant amino acid content of arginine and lysine) and the ability to form an amphipathic structure. It is the cationic character that allows the peptides to selectively recognize prokaryotic cell membranes from eukaryotic ones, based on differences in composition. The bacterial membranes are strongly electronegative under physiological conditions due to the presence of negatively charged phospholipid molecules of cardiolipin, phosphatidyl glycerol and phosphatidylserine. This predominantly physical mechanism of action of AMPs is associated with a very low risk of developing resistant bacterial strains compared to classical antibiotics. Due to their wide-spectrum effect and minimal cytotoxicity to mammalian cells, these peptides are considered to be very promising new-generation drugs that could replace existing therapeutics, especially in the treatment of diseases caused by multi-resistant strains of microorganisms. Three major groups of AMPs occur in the human organism, namely defensins, histatins and cathelicidins. Cathelicidins are mammalian antimicrobial peptides that are stored in neutrophil granulocytes and macrophage cells from where they are released after leukocyte activation. Cathelicidins are synthesized in the form of inactive prepropeptides consisting of a variable N-terminal signal sequence responsible for subcellular distribution, a relatively conserved cathelin domain with a pronounced interspecies homology, and a C-terminal antimicrobial domain characterized by a high intra-and inter-species variability. In humans, only one gene encoding a protein from the cathelicidin group, namely the so-called *CAMP* gene (Cathelicidin AntiMicrobial Peptide), has been described. The *CAMP* gene is located on chromosome 3, is approximately 2 kb in size and contains 4 exons. The gene product is an antimicrobial hCAP-18 protein, and a proteolytic cleavage of its C-terminal end produces an antimicrobial peptide LL-37 (Gudmundsson GH, Agerberth B, Odeberg J, Bergman T, Olsson B, Salcedo R. (1996), Eur J Biochem. 238:325-32). The primary structure of LL-37 is composed of 37 amino acid residues and is responsible for the cationic character of the peptide with a total charge of +6, a size of 4.5 kDa and an isoelectric point of 10.6. LL-37 inhibits the formation of bacterial biofilms and prevents infections of viral (Currie SM, Findlay EG, McHugh BJ, Mackellar A, Man T, Macmillan D, Wang H, Fitch PM, Schwarze J, Davidson DJ. (2013), PLoS One. 8:e73659) or fungal (Ordonez SR, Amarullah IH, Wubbolts RW, Veldhuizen EJ, Haagsman HP. (2014), Antimicrob Agents Chemother. 58:2240-8) origin. Aside from the antimicrobial effect, the peptide inhibits tumor growth (Okumura K, Itoh A, Isogai E, Hirose K, Hosokawa Y, Abiko Y, Shibata T, Hirata M, Isogai H. (2004), Cancer Lett. 30;212:185-94), induces mastocyte chemotaxis (Niyonsaba F, Iwabuchi K, Someya A, Hirata M, Matsuda H, Ogawa H, Nagaoka I. (2002), Immunology. 106:20-6), cell apoptosis (Barlow PG, Li Y, Wilkinson TS, Bowdish DM, Lau YE, Cosseau C, Haslett C, Simpson AJ, Hancock RE, Davidson DJ. (2006), J Leukoc Biol. 80:509-20) or positively affects wound healing processes by stimulating antogenesis and epithelial renewal (Carretero M, Escámez MJ, Garcia M, Duarte B, Holguin A, Retamosa L, Jorcano JL, Rio MD, Larcher F. (2008), J Invest Dermatol. 128:223-36). This multifunctional molecule or its derivatives possess a significant potential for practical commercial applications in pharmaceutical or cosmetic sector. LL-37 could, for example, be useful in the treatment of chronic polymicrobial infections, by means of local topical application to the injured area of the skin (Duplantier AJ, van Hoek ML. (2013), Front Immunol. 4:143). In 2014, the results of a clinical study were published in which a topical application of the LL-37 peptide was evaluated as a safe means for the treatment of patients with chronic leg ulcers (Grönberg A, Mahlapuu M, Ståhle M, Whately-Smith C, Rollman O. (2014), Wound Repair Regen. 22:613-21). At present, the LL-37 peptide undergoes clinical trials as an immunomodulator promoting melanoma treatment by local intratumoral injections (https://clinicaltrials.gov/ct2/show/NCT02225366, staženo dne 14.4.2017).

The main stumbling stone for the commercial use of AMPs, including cathelicidins, is the high production price of the product, which, for example, is in the order of hundreds to thousands of Euros per mg for LL-37 prepared by chemical synthesis, depending on purity. From a pricing perspective, recombinant technologies with prokaryotic or eukaryotic expression systems offer a tremendous potential. Bacterial and yeast expression systems generally provide high yields of protein products and a relatively simple manipulation requirements. However, the use of these microbial expression systems for the production of AMPs is not appropriate precisely because of their antimicrobial properties, which can lead to the killing of the host cell. A disadvantage of insect and mammalian cell suspensions is the high price associated with relatively complex cultivation conditions and the risk of contamination by prions, viruses or toxins. On the other hand, the use of intact plants for the production of AMPs, the so-called plant molecular farming, offers a number of advantages over other systems: 1) production is usually less financially demanding, 2) there is no risk of contamination by oncogenic DNA, endotoxins or human pathogens, 3) the production can be converted into an industrial scale, 4) in most cases, relatively simple peptide purification methods can be used. Recently, the interest of a number of scientific groups in the use of barley as a host for the production of various commercially applicable substances has grown considerably for several reasons. On the one hand, there is an effective method of genetic modification using the supervirulent strain of *Agrobacterium tumefaciens.* Compared to other cereals, barley has a simple diploid genome that guarantees the stability of the transgenic line. The barley plants are selffertilizing, and this eliminates the risk of leakage of the transgene into the surrounding environment, which ranks barley among GRAS certified plants (Generally Recognized as Safe) by the Food and Drug Administration (FDA).

Despite all the above-mentioned benefits of plant systems including barley, AMPs expression in plant systems remains problematic as it often involves low yields or a loss of antimicrobial activity due to the susceptibility of the AMPs to proteolytic degradation (Moncla BJ, Pryke K, Rohan LC, Graebing PW. (2011), Adv Biosci Biotechnol. 2:404-408). Furthermore, AMPs may have toxic effects towards the host plant and cause its abnormal, undesirable phenotype including sterility (Nadal A, Montero M, Company N, Badosa E, Messeguer J, Montesinos L, Montesinos E, Pla M. (2012), BMC Plant Biol. 12: 159). The amount, stability and potential phytotoxicity of the peptides produced in plants is therefore influenced by a number of factors including the selection of the plant production system and of the target tissue, the selection of suitable fusion peptide sequences responsible for intracellular targeting of the peptide, its stability and biological activity.

Based on previously published results, the gene for cathelicidin *LL-37* or its variant was introduced into three different plants, namely Chinese cabbage (*Brassica rapa* var. *chinensis*) (Jung YJ, Lee SY, Moon YS, Kang KK. (2012), Plant Biotechnol Rep. 6:39-46), tomato (*Solanum lycopersicum)* (Jung YJ. (2013), Biotechnol. Bioprocess Eng. 18, 615-24) or rice (*Oryza sativa* L. var. Japonica cv. Dongjinbyeo) (Lee IH, Jung YJ, Cho YG, Nou IS, Huq MA, Nogoy FM, Kang KK. (2017), PLoS One.12:e0172936). Expression of *LL-37* was always driven by a constitutive promoter and the product was located in extracellular space. In all three cases, LL-37 was not produced for molecular farming, but for plant bioprotection against selected phytopathogenic microorganisms. Although expression was regulated by a constitutive promoter, the authors always demonstrated the presence of the peptide by molecular biological methods only in leaves and not in other plant tissues. Moreover, the peptide concentration in the leaves was not determined. Constitutive expression does not seem to be suitable for the production and subsequent isolation of AMP because it can lead to reduced plant vitality, relatively low peptide concentration in the tissues and, moreover, peptides are poorly obtainable from vegetative tissues such as leaves, due to high concentrations of pigments and other secondary metabolites, which typically interfere with the extraction and purification process. Furthermore, vegetative tissues are unable to accumulate recombinant proteins over a long period of time because they lack the ability to switch to dormancy and contain a large amount of proteolytic enzymes involved in product degradation. For this reason, the plant material must be removed and immediately processed at a specific time just when the product is accumulated to the highest degree.

Cathelicidins in an active form are commercially available only in a synthetic form. Cathelicidins prepared for heterologous expression in *E*. *coli* can also be purchased on the market, but they are always in the form of inactive precursors.

### Disclosure of the Invention

The present invention is based on the use of barley for the production of antimicrobial peptides such that the peptide is expressed and/or accumulated in barley grains. Due to the low content of phenolic substances and a large number of protease inhibitors, barley grain is a biochemically inert medium suitable for targeted peptide expression. Due to the ability of the grain to move into dormancy, this generative organ is also suitable for long-term storage of the peptide in bioactive form. Endosperm occupies most of the content of the caryopsis, and majority of storage proteins are stored therein, while gene promoters of the abundant residual protein can be advantageously used for the targeted expression of a transgene. The present invention describes the preparation of an active form of cathelicidin which can be easily isolated from barley endosperm e.g. in the form of proteinaceous bodies. Thus the invention is a technology which solves the above mentioned drawbacks associated with the use of heterologous systems for the production of antimicrobial substances, through selecting key parameters affecting the level of transcription as well as the amount, stability, toxicity and biological activity of the resulting recombinant product. This innovative and advanced technology could find application in the field of plant molecular farming in the form of production of substances containing an active antimicrobial peptide for pharmaceutical, cosmetic or industrial sectors.

Object of the present invention is thus a method of production of barley plants producing antimicrobial peptides, comprising the step of insertion of a nucleotide sequence containing
- a sequence encoding an N-terminal signal peptide from a plant other than barley for translocation of a peptide through endoplasmatic reticulum, selected from N-terminal transient signal peptide of maize cytokinin oxidase/dehydrogenase 1 and N-terminal signal peptide of rice chitinase 1, and
- a sequence encoding at least one antimicrobial peptide selected from a group consisting of cathelicidins, defensins and magainins,

into the genome of a barley plant;
and wherein the nucleotide sequence is under the control of a grain-specific or a constitutive promoter selected from a ubiquitin promoter, a B1 hordein promoter, a trypsin inhibitor barley promoter, an oat globulin promoter.

Preferably, the nucleotide sequence further contains a sequence encoding a C-terminal sequence for the retention of a peptide on endoplasmatic reticulum, such as the sequence KDEL (SEQ ID NO. 7).

Preferably, the nucleotide sequence further contains a sequence encoding at least one stabilization and/or purification tag, such as a histidine tag, a maltose-binding protein, a restriction site recognized by a protease such as enterokinase. Preferably, such sequence may be selected from a group consisting of sequences encoding maltose-binding protein (MBP, SEQ ID NO. 8), histidine tag (6xHis; 8×His, SEQ ID NO. 9 and 10), SUMOstar (SEQ ID NO. 11), putative barley SUMO sequence (SEQ ID NO. 12), DAMP4 protein (SEQ ID NO. 13) and/or flexible binding sequence (GGGGS)₂ (SEQ ID NO. 14).

Preferably, the antimicrobial peptide is a cathelicidin; more preferably, the antimicrobial peptide is a recombinant human cathelicidin LL-37 (PDB: 2K6O_A).

In other embodiments, the antimicrobial peptide may be beta-defensin 2 (HBD2) or pexiganan (PEX).

The N-terminal signal peptide is N-terminal transient signal peptide of maize cytokinin oxidase/dehydrogenase 1 (ZmCKX1sp; GenBank NM_001112121.1; SEQ ID NO. 5) or N-terminal signal peptide of rice chitinase 1 (OsCht1sp; GenBank: D16221.1, SEQ ID NO. 6).

Preferably, the nucleotide sequence for insertion into a barley plant is selected from the group of sequences having at least 80%, more preferably at least 90%, identity with the sequences SEQ ID NO. 1, SEQ ID NO. 2, SEQ ID NO 3., SEQ ID NO 4; more preferably, the nucleotide sequence is selected from the group of sequences SEQ ID NO. 1, SEQ ID NO. 2, SEQ ID NO 3., SEQ ID NO 4.

The percentage of identity as mentioned herein can be achieved by replacement of nucleotides by other nucleotides, in particular by other nucleotides resulting in a codon encoding the same amino acid (synonymous codon), or by extension or shortening of the sequence. The herein mentioned percentages of identities are typically at least 80%, preferably at least 90%, more preferably at least 95%.

Preferably, the nucleotide sequence is inserted into the barley plant using the bacterium *Agrobacterium tumefaciens* or microprojectile bombardment transformation (microprojectile transformation) techniques. Said techniques were successfully used for insertion of transgenes into a number of varieties of barley, including the Golden Promise, Baronesse, Colter, Conlon, Crystal, Drummmond, Foster, 90Ab321 varieties.

It was found within the framework of the present invention that in order to achieve an increased accumulation of the antimicrobial peptide such as LL-37 in genetically modified barley plants, it is particularly advantageous to use the N-terminal transient signal peptide of maize cytokinin oxidase/dehydrogenase 1 (herein referred to as ZmCKX1sp; GenBank NM_001112121.1) which controls the translocation of the peptide through endoplasmatic reticulum. In another preferred embodiment, N-terminal signal peptide of rise chitinase 1 (herein referred to as OsCht1sp; GenBank: D16221.1) can be used for this purpose.

The combination of a N-terminal signal peptide together with the C-terminal sequence for retention of the peptide on endoplasmatic reticulum (KDEL) has a further stabilizing effect on accumulation of the antimicrobial peptide.

Furthermore, it was found that for purification using affinity purification methods, it is particularly advantageous to use the maltose-binding protein (MBP), the histidine tag (6x His, 8x His), and/or the flexible binding sequence (GGGGS)₂; this allows an effective separation of functional domains and increases the product stability. In order to increase the peptide stability and to purify the product, the DAMP4 protein which is a biosurfactant possessing thermostable properties (up to 90 °C) and is soluble in environments with high salt concentrations. These unique properties may be used in the isolation and purification process. In order to separate the recombinant product from other undesirable protein or peptide fractions contained in the extract, the artificial sequence SUMOstar (small ubiquitin like modifier) can be used. SUMOstar has a structure similar to ubiquitin, but prevents subsequence ubiquitinylation and thus prevents the proteolysis of the recombinant peptide in the host tissue. Alternatively, putative barley SUMO sequence (HvSUMO) can be used, which was selected from the database of raw barley genome based on its similarity to the SUMOstar sequence (http://webblast.ipk-gatersleben.de/barley/).

In the framework of the present invention, it was also found that the amount of the antimicrobial peptide, in particular of LL-37, accumulated in the grain under the control of barley endosperm-specific promoter for B1 hordein protein (GenBank: X87232.1) is higher than the amount of the peptide accumulated in the grain under the control of constitutive maize ubiquitin promoter.

Object of the present invention is further an expression cassette comprising a promoter, a nucleotide sequence containing a gene encoding an antimicrobial peptide and a terminator. The nucleotide sequence contains the encoding sequences as described above. The promoter is a grain-specific promoter or a constitutive promoter selected from a ubiquitin promoter, B1 hordein promoter, trypsin inhibitor barley promoter, oat globulin promoter. The terminator sequence is preferably a nopaline synthase terminator (Nos terminator).

In one preferred embodiment, the expression cassette contains: barley B1 hordein promoter::ZmCKX1sp_LL-37_KDEL::Nos terminator and has a sequence having at least 80% identity, preferably at least 90% identity, more preferably at least 95% identity with the sequence SEQ ID NO. 15.

In one preferred embodiment, the expression cassette contains: maize ubiquitin promoter::ZmCKX1sp_LL-37_KDEL::Nos terminator and has a sequence having at least 80% identity, preferably at least 90% identity, more preferably at least 95% identity with the sequence SEQ ID NO. 16.

In one preferred embodiment, the expression cassette contains: barley B1 hordein promoter::ZmCKX1sp_(GGGGS)₂_6xHis_(GGGGS)₂_LL-37::Nos terminator and has a sequence having at least 80% identity, preferably at least 90% identity, more preferably at least 95% identity with the sequence SEQ ID NO. 17.

In one preferred embodiment, the expression cassette contains: maize ubiquitin promoter::ZmCKX1sp_(GGGGS)_{2_}6xHis_ (GGGGS)₂_LL-37::Nos terminator and has a sequence having at least 80% identity, preferably at least 90% identity, more preferably at least 95% identity with the sequence SEQ ID NO. 18.

In one preferred embodiment, the expression cassette contains: barley B1 hordein promoter::ZmCKX1sp_6xHis_MBP_LL-37_KDEL::Nos terminator and has a sequence having at least 80% identity, preferably at least 90% identity, more preferably at least 95% identity with the sequence SEQ ID NO. 19.

In one preferred embodiment, the expression cassette contains: maize ubiquitin promoter::ZmCKX1sp_6xHis_MBP_LL-37_KDEL::Nos terminator and has a sequence having at least 80% identity, preferably at least 90% identity, more preferably at least 95% identity with the sequence SEQ ID NO. 20.

In one preferred embodiment, the expression cassette contains: barley B1 hordein promoter::OsCht1sp_6xHis_LL-37::Nos terminator and has a sequence having at least 80% identity, preferably at least 90% identity, more preferably at least 95% identity with the sequence SEQ ID NO. 21.

In an embodiment not part of the invention, the expression cassette contains: oat globulin promoter AsGLO1p::8xHis_HBD2_KDEL::Nos terminator and has a sequence having at least 80% identity, preferably at least 90% identity, more preferably at least 95% identity with the sequence SEQ ID NO. 22.

In an embodiment not part of the invention, the expression cassette contains: trypsin inhibitor barley promoter TIp::PEX_KDEL::Nos terminator and has a sequence having at least 80% identity, preferably at least 90% identity, more preferably at least 95% identity with the sequence SEQ ID NO. 23.

A further object of the present invention is the use of the said expression cassettes for the production of barley plants producing a recombinant antimicrobial peptide, preferably human cathelicidin LL-37, using the bacterium *Agrobacterium tumefaciens* or microprojectile bombardment transformation (microprojectile transformation) techniques.

A yet further object of the present invention is a barley plant containing in its genome the nucleotide sequence described herein above.

Chimeric peptidic variants of LL-37 possessing antimicrobial effects against gram-negative (G-) bacteria may be produced by the method of the present invention. Preferably, the chimeric peptidic variants of LL-37 are polypeptides selected from the groups consisting of sequences SEQ ID NO 24., SEQ ID NO 25. and SEQ ID NO 26. In the framework of the present invention it was found that to achieve and maintain the antimicrobial effects of LL-37, it is not necessary to cleave the fusion sequences by means of expensive proteases and thus the antimicrobial peptide may be produced at a lower cost.
**Nucleotide sequence encoding the peptide ZmCKX1sp_LL-37_KDEL (59 amino acids, SEQ ID NO 1.)** 
ATGGCCGTTGTTTACTACCTCCTCCTCGCCGGCCTCATTGCCTGCTCTCACGCCCTCCTCGGC
GATTTCTTCCGCAAGTCCAAGGAGAAGATCGGCAAGGAGTTCAAGAGGATCGTCCAGAGGA
TCAAGGACTTCCTCCGCAACCTCGTGCCAAGGACCGAGAGCAAGGACGAGCTTTGA
**Nucleotide sequence encoding the peptide ZmCKX1sp_(GGGGS)_{2_}6xHis_ (GGGGS)₂_LL-37 (86 amino acids, SEQ ID NO 2.)** 
ATGGCCGTTGTTTACTACCTCCTCCTCGCCGGCCTCATTGCCTGCTCCCACGCTGGCGGCGG
CGGCTCCGGCGGCGGGGGCAGCCACCATCACCATCATCATGGCGGGGGCGGCAGCGGGGG
GGGCGGCTCAGATGACGACGACAAGCTCCTTGGCGACTTCTTCCGCAAGTCCAAGGAGAAG
ATCGGCAAGGAGTTCAAGAGGATCGTCCAGAGGATCAAGGACTTCCTGAGGAACCTCGTGC
CGAGGACCGAGTCTTGA
**Nucleotide sequence encoding the protein ZmCKX1sp_6xHis_MBP_LL-37_KDEL (452 amino acids, SEQ ID NO 3.)** 
ATGGCCGTGGTGTACTACCTCCTCCTCGCCGGCCTGATCGCCTGCTCCCACGCGCACCACCA
CCATCACCATAAGATCGAGGAGGGCAAGCTCGTCATCTGGATCAACGGCGACAAGGGCTAC
AACGGCCTCGCCGAGGTGGGCAAGAAGTTCGAGAAGGACACCGGCATCAAGGTGACCGTC
GAGCACCCAGATAAGCTGGAGGAGAAGTTCCCGCAGGTCGCCGCCACCGGCGACGGCCCG
GATATCATCTTCTGGGCCCACGACAGGTTCGGCGGCTACGCCCAGTCCGGCCTCCTCGCCGA
GATCACCCCGGACAAGGCCTTCCAGGATAAGCTCTACCCGTTCACCTGGGACGCCGTGAGG
TACAACGGCAAGCTCATTGCCTACCCGATTGCCGTCGAGGCCCTCTCCCTGATCTACAACAA
 
GGACCTCCTCCCGAACCCGCCCAAGACCTGGGAGGAGATCCCGGCCCTCGACAAGGAGCTG
AAGGCCAAGGGCAAGTCCGCCCTCATGTTCAACCTCCAGGAGCCGTACTTCACCTGGCCAC
TCATTGCGGCCGACGGGGGGTACGCCTTCAAGTACGAGAACGGCAAGTACGACATCAAGGA
CGTGGGCGTGGACAACGCTGGCGCCAAGGCGGGCCTCACCTTCCTCGTGGATCTCATCAAG
AACAAGCACATGAACGCCGACACCGACTACTCGATCGCCGAGGCCGCCTTCAACAAGGGCG
AGACCGCCATGACCATTAACGGCCCGTGGGCCTGGTCCAACATCGACACCTCTAAGGTGAA
CTACGGCGTGACCGTGCTCCCGACCTTCAAGGGCCAGCCGTCCAAGCCGTTCGTGGGCGTG
CTCTCCGCCGGCATCAACGCCGCCTCCCCGAACAAGGAGCTGGCCAAGGAGTTCCTGGAGA
ACTACCTCCTCACCGACGAGGGCCTGGAGGCCGTGAACAAGGACAAGCCGCTCGGCGCCGT
GGCCCTCAAGTCCTACGAGGAGGAGCTGGCGAAGGACCCGAGGATCGCCGCGACCATGGA
GAACGCCCAGAAGGGCGAGATCATGCCGAACATCCCGCAGATGTCCGCCTTCTGGTACGCC
GTCAGGACCGCCGTGATTAACGCCGCCAGCGGCAGGCAGACCGTGGACGAGGCCCTGAAG
GACGCCCAGACCAACTCTTCCTCGAACAATAACAATAACAACAATAATAACAACCTCGGCG
ACGACGACGACAAGCTCCTCGGCGACTTCTTCCGCAAGTCCAAGGAGAAGATCGGCAAGGA
GTTCAAGAGGATCGTCCAGAGGATCAAGGACTTCCTCCGCAACCTCGTGCCAAGGACCGAG
TCCAAGGATGAGCTGTGA
**Nucleotide sequence encoding the peptide OsCht1sp_6xHis_LL-37 (68 amino acids, SEQ ID NO 4.)** 
ATGAGGGCTCTCGCCGTGGTGGTGGTCGCCACCGCCTTCGCTGTGGTGGCCGTGAGGGGCC
ACCATCACCATCATCATGACGACGACGATAAGCTCCTCGGCGACTTCTTCCGCAAGTCCAAG
GAGAAGATCGGCAAGGAGTTCAAGAGGATCGTCCAGAGGATCAAGGACTTCCTGAGGAAC
CTCGTGCCGAGGACCGAGTCTTGA
**Nucleotide sequence encoding the peptide ZmCKX1sp (18 amino acids, SEQ ID NO. 5)**
   ATGGCCGTTGTTTACTACCTCCTCCTCGCCGGCCTCATTGCCTGCTCTCACGCCTGA
**Nucleotide sequence encoding the peptide OsCht1sp (20 amino acids, SEQ ID NO. 6)** 
ATGAGGGCTCTCGCCGTGGTGGTGGTCGCCACCGCCTTCGCTGTGGTGGCCGTGAGGGGCT
GA
**Nucleotide sequence encoding the peptide KDEL (5 amino acids, SEQ ID NO. 7)**
   ATGAAGGATGAGCTGTGA
Nucleotide sequence encoding the protein MBP (383 amino acids, SEQ ID NO. 8) 
ATGAAGATCGAGGAGGGCAAGCTCGTCATCTGGATCAACGGCGACAAGGGCTACAACGGC
CTCGCCGAGGTGGGCAAGAAGTTCGAGAAGGACACCGGCATCAAGGTGACCGTCGAGCAC
CCAGATAAGCTGGAGGAGAAGTTCCCGCAGGTCGCCGCCACCGGCGACGGCCCGGATATCA
TCTTCTGGGCCCACGACAGGTTCGGCGGCTACGCCCAGTCCGGCCTCCTCGCCGAGATCACC
CCGGACAAGGCCTTCCAGGATAAGCTCTACCCGTTCACCTGGGACGCCGTGAGGTACAACG
GCAAGCTCATTGCCTACCCGATTGCCGTCGAGGCCCTCTCCCTGATCTACAACAAGGACCTC
CTCCCGAACCCGCCCAAGACCTGGGAGGAGATCCCGGCCCTCGACAAGGAGCTGAAGGCCA
AGGGCAAGTCCGCCCTCATGTTCAACCTCCAGGAGCCGTACTTCACCTGGCCACTCATTGCG
GCCGACGGGGGGTACGCCTTCAAGTACGAGAACGGCAAGTACGACATCAAGGACGTGGGC
GTGGACAACGCTGGCGCCAAGGCGGGCCTCACCTTCCTCGTGGATCTCATCAAGAACAAGC
ACATGAACGCCGACACCGACTACTCGATCGCCGAGGCCGCCTTCAACAAGGGCGAGACCGC
CATGACCATTAACGGCCCGTGGGCCTGGTCCAACATCGACACCTCTAAGGTGAACTACGGC
GTGACCGTGCTCCCGACCTTCAAGGGCCAGCCGTCCAAGCCGTTCGTGGGCGTGCTCTCCGC
CGGCATCAACGCCGCCTCCCCGAACAAGGAGCTGGCCAAGGAGTTCCTGGAGAACTACCTC
CTCACCGACGAGGGCCTGGAGGCCGTGAACAAGGACAAGCCGCTCGGCGCCGTGGCCCTCA
AGTCCTACGAGGAGGAGCTGGCGAAGGACCCGAGGATCGCCGCGACCATGGAGAACGCCC
AGAAGGGCGAGATCATGCCGAACATCCCGCAGATGTCCGCCTTCTGGTACGCCGTCAGGAC
CGCCGTGATTAACGCCGCCAGCGGCAGGCAGACCGTGGACGAGGCCCTGAAGGACGCCCA
GACCAACTCTTCCTCGAACAATAACAATAACAACAATAATAACAACCTCGGCTGA
Nucleotide sequence encoding 6xHis (7 amino acids, SEQ ID NO. 9)
   ATGCACCATCACCATCATCATTGA
Nucleotide sequence encoding 8xHis (9 amino acids, SEQ ID NO. 10)
   ATGCACCATCACCATCATCATCATCATTGA
Nucleotide sequence encoding SUMOstar (101 amino acids, SEQ ID NO. 11) 
ATGTCCCTTCAGGACTCCGAGGTGAACCAGGAGGCCAAGCCAGAGGTTAAGCCTGAGGTGA
AGCCCGAGACCCACATCAACCTCAAGGTGTCCGACGGCTCCTCCGAGATCTTCTTCAAGATC
AAGAAGACCACCCCGCTCAGGCGCCTCATGGAGGCCTTCGCCAAGAGGCAGGGCAAGGAG
ATGGATTCCCTCACCTTCCTCTACGACGGCATCGAGATCCAGGCCGATCAGACCCCAGAGG
 
ACCTCGACATGGAGGACAACGACATCATTGAGGCCCACCGCGAGCAGATTGGCGGCCTTTG
A
**Nucleotide sequence encoding HvSUMO (96 amino acids, SEQ ID NO. 12)** 
ATGTCTGGCGTTACCGCCGACGAGGATAAGAAGCCAGCCGGCGACGGCGGCGCTCATA
TCAACCTCAAGGTTAAGGGCCAGGACGGCAACGAGGTGTTCTTCAGGATCAAGAGGTCCAC
CCAGCTCAAGAAGCTCATGAACGCCTACTGCGACAGGCAGTCCGTGGACCTCAACTCGATC
GCCTTCCTGTTCGATGGCAGGCGCCTCAGGGGCGAGCAGACCCCAGACGAGCTTGAGATGG
AGGAGGGCGACGAGATTGACGCCATGCTTCACCAGACCGGCGGCTGA
**Nucleotide sequence encoding DAMP4 (100 amino acids, SEQ ID NO. 13)** 
ATGGAGCCGTCCATGAAGCAGCTCGCCGATTCCCTCCACCAGCTCGCCAGGCAGGTTTCCA
GGCTTGAGCACGCCGAGCCTAGCATGAAGCAGCTTGCTGACAGCCTCCATCAGCTTGCCCG
CCAGGTGTCACGCCTTGAGCATGCTGAGCCATCGATGAAGCAGCTGGCGGACTCTCTGCAT
CAGCTGGCTAGGCAGGTCAGCAGGCTTGAGCATGCGGAGCCTTCCATGAAGCAGCTTGCGG
ATAGCCTTCACCAGCTGGCGCGGCAGGTTAGCCGCCTTGAGCACGCGGATCCATCTTGA
**Nucleotide sequence encoding (GGGGS)₂ (11 amino acids, SEQ ID NO. 14)**
   ATGGGCGGCGGCGGCTCCGGCGGCGGGGGCAGCTGA
**SEQ ID NO. 15:**
   Positions of individual functional components:
   B1 hordein promoter: 13 bp - 562 bp
   Kozak consensus sequence: 581 bp - 589 bp
   ZmCKX1sp: 590 bp - 640 bp
   LL-37: 641 bp - 751 bp
   KDEL: 752 bp - 763 bp
   Nos terminator: 791 bp - 1074 bp 
TTTAAAGTCGACGTGCAGGTGTATGAGTCATTGTTATGATCTATAGGTGTCAGTTTATCTTAT
CATCTGGGTGATCAATACAGGCCCAGGTTTTATAAAAACCAGTCGAGTCGAGAAGAACCGT
CCACATGTAAAGCTTTAACAACCCACACATTGATTGCAACTTAGTCCTACACAAGTTTTCCA
TTCTTGTTTCAGGCTAACAACCTATACAAGGTTCCAAAATCATGCAAAAGTGATGCTAGGTT
GATAATGTGTGACATGTAAAGTGAATAAGGTGAGTCATGCATACCAAACCTCGGGATTTCT
 
ATACTTTGTGTATGATCATATGCACAACTAAAAGGCAACTTTGATTATCAATTGAAAAGTAC
CGCTTGTAGCTTGTGCAACCTAACACAATGTCCAAAAATCCATTTGCAAAAGCATCCAAAC
ACAATTGTTAAAGCTGTTCAAACAAACAAAGAAGAGATGAAGCCTGGCTACTATAAATAGG
CAGGTAGTATAGAGATCTACACAAGCACAAGCATCAAAACCAAGAAACACTAGTTAACACC
AATCCACTGTCGACGGTACCGGATCCGCCACCATGGCCGTTGTTTACTACCTCCTCCTCGCC
GGCCTCATTGCCTGCTCTCACGCCCTCCTCGGCGATTTCTTCCGCAAGTCCAAGGAGAAGAT
CGGCAAGGAGTTCAAGAGGATCGTCCAGAGGATCAAGGACTTCCTCCGCAACCTCGTGCCA
AGGACCGAGAGCAAGGACGAGCTTTGACTCGAGCCTTGGTCTAGAGAGCTCAGTCAAGCAG
GATCGTTCAAACATTTGGCAATAAAGTTTCTTAAGATTGAATCCTGTTGCCGGTCTTGCGAT
GATTATCATATAATTTCTGTTGAATTACGTTAAGCATGTAATAATTAACATGTAATGCATGA
CGTTATTTATGAGATGGGTTTTTATGATTAGAGTCCCGCAATTATACATTTAATACGCGATA
GAAAACAAAATATAGCGCGCAAACTAGGATAAATTATCGCGCGCGGTGTCATCTATGTTAC
TAGATCGACCGGCATGCAAGCTGATATGAGCTCGCATGCGCGGCCGCGATATC
**SEQ ID NO. 16:**
   Positions of individual functional components:
   Maize ubiquitin promoter: 1 bp - 1987 bp
   Kozak consensus sequence: 2056 bp - 2064 bp
   ZmCKX1sp: 2065 bp - 2115 bp
   LL-37: 2116 bp - 2226 bp
   KDEL: 2227 bp - 2238 bp
   Nos terminator: 2335 bp - 2618 bp 
GTGCAGCGTGACCCGGTCGTGCCCCTCTCTAGAGATAATGAGCATTGCATGTCTAAGTTATA
AAAAATTACCACATATTTTTTTTGTCACACTTGTTTGAAGTGCAGTTTATCTATCTTTATACA
TATATTTAAACTTTACTCTACGAATAATATAATCTATAGTACTACAATAATATCAGTGTTTTA
GAGAATCATATAAATGAACAGTTAGACATGGTCTAAAGGACAATTGAGTATTTTGACAACA
GGACTCTACAGTTTTATCTTTTTAGTGTGCATGTGTTCTCCTTTTTTTTTGCAAATAGCTTCAC
CTATATAATACTTCATCCATTTTATTAGTACATCCATTTAGGGTTTAGGGTTAATGGTTTTTA
TAGACTAATTTTTTTAGTACATCTATTTTATTCTATTTTAGCCTCTAAATTAAGAAAACTAAA
ACTCTATTTTAGTTTTTTTATTTAATAATTTAGATATAAAATAGAATAAAATAAAGTGACTA
AAAATTAAACAAATACCCTTTAAGAAATTAAAAAAACTAAGGAAACATTTTTCTTGTTTCGA
GTAGATAATGCCAGCCTGTTAAACGCCGTCGACGAGTCTAACGGACACCAACCAGCGAACC
AGCAGCGTCGCGTCGGGCCAAGCGAAGCAGACGGCACGGCATCTCTGTCGCTGCCTCTGGA
 
CCCCTCTCGAGAGTTCCGCTCCACCGTTGGACTTGCTCCGCTGTCGGCATCCAGAAATTGCG
TGTCGGACGGCAGACGTGAGCCGGCACGGCAGGCGGCCTCCTCCTCCTCTCACGGCACCGG
CAGCTACGGGGGATTCCTTTCCCACCGCTCCTTCGCTTTCCCTTCCTCGCCCGCCGTAATAAA
TAGACACCCCCTCCACACCCTCTTTCCCCAACCTCGTGTTGTTCGGAGCGCACACACACACA
ACCAGATCTCCCCCAAATCCACCCGTCGGCACCTCCGCTTCAAGGTACGCCGCTCGTCCTCC
CCCCCCCCCCCTCTCTACCTTCTCTAGATCGGCGTTCCGGTCCATGGTTAGGGCCCGGTAGTT
CTACTTCTGTTCATGTTTGTGTTAGATCCGTGTTTGTGTTAGATCCGTGCTGCTAGCGTTCGT
ACACGGATGCGACCTGTACGTCAGACACGTTCTGATTGCTAACTTGCCAGTGTTTCTCTTTG
GGGAATCCTGGGATGGCTCTAGCCGTTCCGCAGACGGGATCGATTTCATGATTTTTTTTTGT
TTCGTTGCATAGGGTTTGGTTTGCCCTTTTCCTTTATTTCAATATATGCCGTGCACTTGTTTGT
CGGGTCATCTTTTCATGCTTTTTTTTGTCTTGGTTGTGATGATGTGGTCTGGTTGGGCGGTCG
TTCTAGATCGGAGTAGAATTAATTCTGTTTCAAACTACCTGGTGGATTTATTAATTTTGGATC
TGTATGTGTGTGCCATACATATTCATAGTTACGAATTGAAGATGATGGATGGAAATATCGAT
CTAGGATAGGTATACATGTTGATGCGGGTTTTACTGATGCATATACAGAGATGCTTTTTGTT
CGCTTGGTTGTGATGATGTGGTGTGGTTGGGCGGTCGTTCATTCGTTCTAGATCGGAGTAGA
ATACTGTTTCAAACTACCTGGTGTATTTATTAATTTTGGAACTGTATGTGTGTGTCATACATC
TTCATAGTTACGAGTTTAAGATGGATGGAAATATCGATCTAGGATAGGTATACATGTTGATG
TGGGTTTTACTGATGCATATACATGATGGCATATGCAGCATCTATTCATATGCTCTAACCTT
GAGTACCTATCTATTATAATAAACAAGTATGTTTTATAATTATTTTGATCTTGATATACTTGG
ATGATGGCATATGCAGCAGCTATATGTGGATTTTTTTAGCCCTGCCTTCATACGCTATTTATT
TGCTTGGTACTGTTTCTTTTGTCGATGCTCACCCTGTTGTTTGGTGTTACTTCGCCCATCACA
AGTTTGTACAAAAAAGCAGGCTGGCGCCGGAACCAATTCAGTCGACCGTACGGGATCCGCC
ACCATGGCCGTTGTTTACTACCTCCTCCTCGCCGGCCTCATTGCCTGCTCTCACGCCCTCCTC
GGCGATTTCTTCCGCAAGTCCAAGGAGAAGATCGGCAAGGAGTTCAAGAGGATCGTCCAGA
GGATCAAGGACTTCCTCCGCAACCTCGTGCCAAGGACCGAGAGCAAGGACGAGCTTTGACT
CGAGGAATTCGCGGCCGCACTCGAGATATCTAGACCCAGCTTTCTTGTACAAAGTGGTGAT
GGGGGATCCACTAGTTCTAGAATTCGATTGAGTCAAGCAGGATCGTTCAAACATTTGGCAA
TAAAGTTTCTTAAGATTGAATCCTGTTGCCGGTCTTGCGATGATTATCATATAATTTCTGTTG
AATTACGTTAAGCATGTAATAATTAACATGTAATGCATGACGTTATTTATGAGATGGGTTTT
TATGATTAGAGTCCCGCAATTATACATTTAATACGCGATAGAAAACAAAATATAGCGCGCA
AACTAGGATAAATTATCGCGCGCGGTGTCATCTATGTTACTAGATCGACCGGCATGCAAGCT
GATAT
**SEQ ID NO. 17:**
   Positions of individual functional components:
   B1 hordein promoter: 13 bp - 562 bp
   Kozak consensus sequence: 581 bp - 589 bp
   ZmCKX1sp: 590 bp - 640 bp
   (GGGGS)₂: 641 bp - 670 bp, 689 bp - 718 bp
   6xHis: 671 bp - 688 bp
   Enterokinase-recognized restriction site: 719 bp - 733 bp
   LL-37: 734 bp - 844 bp
   Nos terminator: 872 bp - 1155 bp 
TTTAAAGTCGACGTGCAGGTGTATGAGTCATTGTTATGATCTATAGGTGTCAGTTTATCTTAT
CATCTGGGTGATCAATACAGGCCCAGGTTTTATAAAAACCAGTCGAGTCGAGAAGAACCGT
CCACATGTAAAGCTTTAACAACCCACACATTGATTGCAACTTAGTCCTACACAAGTTTTCCA
TTCTTGTTTCAGGCTAACAACCTATACAAGGTTCCAAAATCATGCAAAAGTGATGCTAGGTT
GATAATGTGTGACATGTAAAGTGAATAAGGTGAGTCATGCATACCAAACCTCGGGATTTCT
ATACTTTGTGTATGATCATATGCACAACTAAAAGGCAACTTTGATTATCAATTGAAAAGTAC
CGCTTGTAGCTTGTGCAACCTAACACAATGTCCAAAAATCCATTTGCAAAAGCATCCAAAC
ACAATTGTTAAAGCTGTTCAAACAAACAAAGAAGAGATGAAGCCTGGCTACTATAAATAGG
CAGGTAGTATAGAGATCTACACAAGCACAAGCATCAAAACCAAGAAACACTAGTTAACACC
AATCCACTGTCGACGGTACCGGATCCGCCACCATGGCCGTTGTTTACTACCTCCTCCTCGCC
GGCCTCATTGCCTGCTCCCACGCTGGCGGCGGCTCCGGCGGCGGGGGCAGCCACCATC
ACCATCATCATGGCGGGGGCGGCAGCGGGGGGGGCGGCTCAGATGACGACGACAAGCTCC
TTGGCGACTTCTTCCGCAAGTCCAAGGAGAAGATCGGCAAGGAGTTCAAGAGGATCGTCCA
GAGGATCAAGGACTTCCTGAGGAACCTCGTGCCGAGGACCGAGTCTTGACTCGAGCCTTGG
TCTAGAGAGCTCAGTCAAGCAGGATCGTTCAAACATTTGGCAATAAAGTTTCTTAAGATTGA
ATCCTGTTGCCGGTCTTGCGATGATTATCATATAATTTCTGTTGAATTACGTTAAGCATGTAA
TAATTAACATGTAATGCATGACGTTATTTATGAGATGGGTTTTTATGATTAGAGTCCCGCAA
TTATACATTTAATACGCGATAGAAAACAAAATATAGCGCGCAAACTAGGATAAATTATCGC
GCGCGGTGTCATCTATGTTACTAGATCGACCGGCATGCAAGCTGATATGAGCTCGCATGCGC
GGCCGCGATATC
**SEQ ID NO. 18:**
   Positions of individual functional components:
   Maize ubiquitin promoter: 1 bp - 1987 bp
   Kozak consensus sequence: 2056 bp - 2064 bp
   ZmCKX1sp: 2065 bp - 2115 bp
   (GGGGS)₂: 2116 bp - 2145 bp, 2164 bp - 2193 bp
   6xHis: 2146 bp - 2163 bp
   Enterokinase-recognized restriction site: 2194 bp - 2208 bp
   LL-37: 2209 bp - 2319 bp
   Nos terminator: 2416 bp - 2699 bp 
GTGCAGCGTGACCCGGTCGTGCCCCTCTCTAGAGATAATGAGCATTGCATGTCTAAGTTATA
AAAAATTACCACATATTTTTTTTGTCACACTTGTTTGAAGTGCAGTTTATCTATCTTTATACA
TATATTTAAACTTTACTCTACGAATAATATAATCTATAGTACTACAATAATATCAGTGTTTTA
GAGAATCATATAAATGAACAGTTAGACATGGTCTAAAGGACAATTGAGTATTTTGACAACA
GGACTCTACAGTTTTATCTTTTTAGTGTGCATGTGTTCTCCTTTTTTTTTGCAAATAGCTTCAC
CTATATAATACTTCATCCATTTTATTAGTACATCCATTTAGGGTTTAGGGTTAATGGTTTTTA
TAGACTAATTTTTTTAGTACATCTATTTTATTCTATTTTAGCCTCTAAATTAAGAAAACTAAA
ACTCTATTTTAGTTTTTTTATTTAATAATTTAGATATAAAATAGAATAAAATAAAGTGACTA
AAAATTAAACAAATACCCTTTAAGAAATTAAAAAAACTAAGGAAACATTTTTCTTGTTTCGA
GTAGATAATGCCAGCCTGTTAAACGCCGTCGACGAGTCTAACGGACACCAACCAGCGAACC
AGCAGCGTCGCGTCGGGCCAAGCGAAGCAGACGGCACGGCATCTCTGTCGCTGCCTCTGGA
CCCCTCTCGAGAGTTCCGCTCCACCGTTGGACTTGCTCCGCTGTCGGCATCCAGAAATTGCG
TGTCGGACGGCAGACGTGAGCCGGCACGGCAGGCGGCCTCCTCCTCTCACGGCACCGG
CAGCTACGGGGGATTCCTTTCCCACCGCTCCTTCGCTTTCCCTTCCTCGCCCGCCGTAATAAA
TAGACACCCCCTCCACACCCTCTTTCCCCAACCTCGTGTTGTTCGGAGCGCACACACACACA
ACCAGATCTCCCCCAAATCCACCCGTCGGCACCTCCGCTTCAAGGTACGCCGCTCGTCCTCC
CCCCCCCCCCCTCTCTACCTTCTCTAGATCGGCGTTCCGGTCCATGGTTAGGGCCCGGTAGTT
CTACTTCTGTTCATGTTTGTGTTAGATCCGTGTTTGTGTTAGATCCGTGCTGCTAGCGTTCGT
ACACGGATGCGACCTGTACGTCAGACACGTTCTGATTGCTAACTTGCCAGTGTTTCTCTTTG
GGGAATCCTGGGATGGCTCTAGCCGTTCCGCAGACGGGATCGATTTCATGATTTTTTTTTGT
TTCGTTGCATAGGGTTTGGTTTGCCCTTTTCCTTTATTTCAATATATGCCGTGCACTTGTTTGT
CGGGTCATCTTTTCATGCTTTTTTTTGTCTTGGTTGTGATGATGTGGTCTGGTTGGGCGGTCG
TTCTAGATCGGAGTAGAATTAATTCTGTTTCAAACTACCTGGTGGATTTATTAATTTTGGATC
TGTATGTGTGTGCCATACATATTCATAGTTACGAATTGAAGATGATGGATGGAAATATCGAT
CTAGGATAGGTATACATGTTGATGCGGGTTTTACTGATGCATATACAGAGATGCTTTTTGTT
 
CGCTTGGTTGTGATGATGTGGTGTGGTTGGGCGGTCGTTCATTCGTTCTAGATCGGAGTAGA
ATACTGTTTCAAACTACCTGGTGTATTTATTAATTTTGGAACTGTATGTGTGTGTCATACATC
TTCATAGTTACGAGTTTAAGATGGATGGAAATATCGATCTAGGATAGGTATACATGTTGATG
TGGGTTTTACTGATGCATATACATGATGGCATATGCAGCATCTATTCATATGCTCTAACCTT
GAGTACCTATCTATTATAATAAACAAGTATGTTTTATAATTATTTTGATCTTGATATACTTGG
ATGATGGCATATGCAGCAGCTATATGTGGATTTTTTTAGCCCTGCCTTCATACGCTATTTATT
TGCTTGGTACTGTTTCTTTTGTCGATGCTCACCCTGTTGTTTGGTGTTACTTCGCCCATCACA
AGTTTGTACAAAAAAGCAGGCTGGCGCCGGAACCAATTCAGTCGACCGTACGGGATCCGCC
ACCATGGCCGTTGTTTACTACCTCCTCCTCGCCGGCCTCATTGCCTGCTCCCACGCTGGCGGC
GGCGGCTCCGGCGGCGGGGGCAGCCACCATCACCATCATCATGGCGGGGGCGGCAGCGGG
GGGGGCGGCTCAGATGACGACGACAAGCTCCTTGGCGACTTCTTCCGCAAGTCCAAGGAGA
AGATCGGCAAGGAGTTCAAGAGGATCGTCCAGAGGATCAAGGACTTCCTGAGGAACCTCGT
GCCGAGGACCGAGTCTTGACTCGAGGAATTCGCGGCCGCACTCGAGATATCTAGACCCAGC
TTTCTTGTACAAAGTGGTGATGGGGGATCCACTAGTTCTAGAATTCGATTGAGTCAAGCAGG
ATCGTTCAAACATTTGGCAATAAAGTTTCTTAAGATTGAATCCTGTTGCCGGTCTTGCGATG
ATTATCATATAATTTCTGTTGAATTACGTTAAGCATGTAATAATTAACATGTAATGCATGAC
GTTATTTATGAGATGGGTTTTTATGATTAGAGTCCCGCAATTATACATTTAATACGCGATAG
AAAACAAAATATAGCGCGCAAACTAGGATAAATTATCGCGCGCGGTGTCATCTATGTTACT
AGATCGACCGGCATGCAAGCTGATAT
**SEQ ID NO. 19:**
   Positions of individual functional components:
   B1 hordein promoter: 13 bp - 562 bp
   Kozak consensus sequence: 581 bp - 592 bp
   ZmCKX1sp: 593 bp - 643 bp
   6xHis: 644 bp - 661 bp
   MBP: 662 bp - 1807 bp
   Enterokinase-recognized restriction site: 1808 bp - 1822 bp
   LL-37: 1823 bp - 1933 bp
   KDEL: 1934 bp - 1945 bp
   Nos terminator: 1973 bp - 2256 bp 
TTTAAAGTCGACGTGCAGGTGTATGAGTCATTGTTATGATCTATAGGTGTCAGTTTATCTTAT
CATCTGGGTGATCAATACAGGCCCAGGTTTTATAAAAACCAGTCGAGTCGAGAAGAACCGT
 
CCACATGTAAAGCTTTAACAACCCACACATTGATTGCAACTTAGTCCTACACAAGTTTTCCA
TTCTTGTTTCAGGCTAACAACCTATACAAGGTTCCAAAATCATGCAAAAGTGATGCTAGGTT
GATAATGTGTGACATGTAAAGTGAATAAGGTGAGTCATGCATACCAAACCTCGGGATTTCT
ATACTTTGTGTATGATCATATGCACAACTAAAAGGCAACTTTGATTATCAATTGAAAAGTAC
CGCTTGTAGCTTGTGCAACCTAACACAATGTCCAAAAATCCATTTGCAAAAGCATCCAAAC
ACAATTGTTAAAGCTGTTCAAACAAACAAAGAAGAGATGAAGCCTGGCTACTATAAATAGG
CAGGTAGTATAGAGATCTACACAAGCACAAGCATCAAAACCAAGAAACACTAGTTAACACC
AATCCACTGTCGACGGTACCGGATCCGCGGCAGCGATGGCCGTGGTGTACTACCTCCTCCTC
GCCGGCCTGATCGCCTGCTCCCACGCGCACCACCACCATCACCATAAGATCGAGGAGGGCA
AGCTCGTCATCTGGATCAACGGCGACAAGGGCTACAACGGCCTCGCCGAGGTGGGCAAGAA
GTTCGAGAAGGACACCGGCATCAAGGTGACCGTCGAGCACCCAGATAAGCTGGAGGAGAA
GTTCCCGCAGGTCGCCGCCACCGGCGACGGCCCGGATATCATCTTCTGGGCCCACGACAGG
TTCGGCGGCTACGCCCAGTCCGGCCTCCTCGCCGAGATCACCCCGGACAAGGCCTTCCAGG
ATAAGCTCTACCCGTTCACCTGGGACGCCGTGAGGTACAACGGCAAGCTCATTGCCTACCC
GATTGCCGTCGAGGCCCTCTCCCTGATCTACAACAAGGACCTCCTCCCGAACCCGCCCAAGA
CCTGGGAGGAGATCCCGGCCCTCGACAAGGAGCTGAAGGCCAAGGGCAAGTCCGCCCTCAT
GTTCAACCTCCAGGAGCCGTACTTCACCTGGCCACTCATTGCGGCCGACGGGGGGTACGCCT
TCAAGTACGAGAACGGCAAGTACGACATCAAGGACGTGGGCGTGGACAACGCTGGCGCCA
AGGCGGGCCTCACCTTCCTCGTGGATCTCATCAAGAACAAGCACATGAACGCCGACACCGA
CTACTCGATCGCCGAGGCCGCCTTCAACAAGGGCGAGACCGCCATGACCATTAACGGCCCG
TGGGCCTGGTCCAACATCGACACCTCTAAGGTGAACTACGGCGTGACCGTGCTCCCGACCTT
CAAGGGCCAGCCGTCCAAGCCGTTCGTGGGCGTGCTCTCCGCCGGCATCAACGCCGCCTCC
CCGAACAAGGAGCTGGCCAAGGAGTTCCTGGAGAACTACCTCCTCACCGACGAGGGCCTGG
AGGCCGTGAACAAGGACAAGCCGCTCGGCGCCGTGGCCCTCAAGTCCTACGAGGAGGAGCT
GGCGAAGGACCCGAGGATCGCCGCGACCATGGAGAACGCCCAGAAGGGCGAGATCATGCC
GAACATCCCGCAGATGTCCGCCTTCTGGTACGCCGTCAGGACCGCCGTGATTAACGCCGCC
AGCGGCAGGCAGACCGTGGACGAGGCCCTGAAGGACGCCCAGACCAACTCTTCCTCGAACA
ATAACAATAACAACAATAATAACAACCTCGGCGACGACGACGACAAGCTCCTCGGCGACTT
CTTCCGCAAGTCCAAGGAGAAGATCGGCAAGGAGTTCAAGAGGATCGTCCAGAGGATCAA
GGACTTCCTCCGCAACCTCGTGCCAAGGACCGAGTCCAAGGATGAGCTGTGACTCGAGCCT
TGGTCTAGAGAGCTCAGTCAAGCAGGATCGTTCAAACATTTGGCAATAAAGTTTCTTAAGAT
TGAATCCTGTTGCCGGTCTTGCGATGATTATCATATAATTTCTGTTGAATTACGTTAAGCATG
TAATAATTAACATGTAATGCATGACGTTATTTATGAGATGGGTTTTTATGATTAGAGTCCCG
 
CAATTATACATTTAATACGCGATAGAAAACAAAATATAGCGCGCAAACTAGGATAAATTAT
CGCGCGCGGTGTCATCTATGTTACTAGATCGACCGGCATGCAAGCTGATATGAGCTCGCATG
CGCGGCCGCGATATC
**SEQ ID NO. 20:**
   Positions of individual functional components:
   Maize ubiquitin promoter: 1 bp - 1987 bp
   Kozak consensus sequence: 2056 bp - 2067 bp
   ZmCKX1sp: 2068 bp - 2118 bp
   6xHis: 2119 bp - 2136 bp
   MBP: 2137 bp - 3282 bp
   Enterokinase-recognized restriction site: 3283 bp - 3297 bp
   LL-37: 3298 bp - 3408 bp
   KDEL: 3409 bp - 3420 bp
   Nos terminator: 3517 bp - 3800 bp 
GTGCAGCGTGACCCGGTCGTGCCCCTCTCTAGAGATAATGAGCATTGCATGTCTAAGTTATA
AAAAATTACCACATATTTTTTTTGTCACACTTGTTTGAAGTGCAGTTTATCTATCTTTATACA
TATATTTAAACTTTACTCTACGAATAATATAATCTATAGTACTACAATAATATCAGTGTTTTA
GAGAATCATATAAATGAACAGTTAGACATGGTCTAAAGGACAATTGAGTATTTTGACAACA
GGACTCTACAGTTTTATCTTTTTAGTGTGCATGTGTTCTCCTTTTTTTTTGCAAATAGCTTCAC
CTATATAATACTTCATCCATTTTATTAGTACATCCATTTAGGGTTTAGGGTTAATGGTTTTTA
TAGACTAATTTTTTTAGTACATCTATTTTATTCTATTTTAGCCTCTAAATTAAGAAAACTAAA
ACTCTATTTTAGTTTTTTTATTTAATAATTTAGATATAAAATAGAATAAAATAAAGTGACTA
AAAATTAAACAAATACCCTTTAAGAAATTAAAAAAACTAAGGAAACATTTTTCTTGTTTCGA
GTAGATAATGCCAGCCTGTTAAACGCCGTCGACGAGTCTAACGGACACCAACCAGCGAACC
AGCAGCGTCGCGTCGGGCCAAGCGAAGCAGACGGCACGGCATCTCTGTCGCTGCCTCTGGA
CCCCTCTCGAGAGTTCCGCTCCACCGTTGGACTTGCTCCGCTGTCGGCATCCAGAAATTGCG
TGTCGGACGGCAGACGTGAGCCGGCACGGCAGGCGGCCTCCTCCTCCTCTCACGGCACCGG
CAGCTACGGGGGATTCCTTTCCCACCGCTCCTTCGCTTTCCCTTCCTCGCCCGCCGTAATAAA
TAGACACCCCCTCCACACCCTCTTTCCCCAACCTCGTGTTGTTCGGAGCGCACACACACACA
ACCAGATCTCCCCCAAATCCACCCGTCGGCACCTCCGCTTCAAGGTACGCCGCTCGTCCTCC
CCCCCCCCCCCTCTCTACCTTCTCTAGATCGGCGTTCCGGTCCATGGTTAGGGCCCGGTAGTT
CTACTTCTGTTCATGTTTGTGTTAGATCCGTGTTTGTGTTAGATCCGTGCTGCTAGCGTTCGT
 
ACACGGATGCGACCTGTACGTCAGACACGTTCTGATTGCTAACTTGCCAGTGTTTCTCTTTG
GGGAATCCTGGGATGGCTCTAGCCGTTCCGCAGACGGGATCGATTTCATGATTTTTTTTTGT
TTCGTTGCATAGGGTTTGGTTTGCCCTTTTCCTTTATTTCAATATATGCCGTGCACTTGTTTGT
CGGGTCATCTTTTCATGCTTTTTTTTGTCTTGGTTGTGATGATGTGGTCTGGTTGGGCGGTCG
TTCTAGATCGGAGTAGAATTAATTCTGTTTCAAACTACCTGGTGGATTTATTAATTTTGGATC
TGTATGTGTGTGCCATACATATTCATAGTTACGAATTGAAGATGATGGATGGAAATATCGAT
CTAGGATAGGTATACATGTTGATGCGGGTTTTACTGATGCATATACAGAGATGCTTTTTGTT
CGCTTGGTTGTGATGATGTGGTGTGGTTGGGCGGTCGTTCATTCGTTCTAGATCGGAGTAGA
ATACTGTTTCAAACTACCTGGTGTATTTATTAATTTTGGAACTGTATGTGTGTGTCATACATC
TTCATAGTTACGAGTTTAAGATGGATGGAAATATCGATCTAGGATAGGTATACATGTTGATG
TGGGTTTTACTGATGCATATACATGATGGCATATGCAGCATCTATTCATATGCTCTAACCTT
GAGTACCTATCTATTATAATAAACAAGTATGTTTTATAATTATTTTGATCTTGATATACTTGG
ATGATGGCATATGCAGCAGCTATATGTGGATTTTTTTAGCCCTGCCTTCATACGCTATTTATT
TGCTTGGTACTGTTTCTTTTGTCGATGCTCACCCTGTTGTTTGGTGTTACTTCGCCCATCACA
AGTTTGTACAAAAAAGCAGGCTGGCGCCGGAACCAATTCAGTCGACCGTACGGGATCCGCG
GCAGCGATGGCCGTGGTGTACTACCTCCTCCTCGCCGGCCTGATCGCCTGCTCCCACGCGCA
CCACCACCATCACCATAAGATCGAGGAGGGCAAGCTCGTCATCTGGATCAACGGCGACAAG
GGCTACAACGGCCTCGCCGAGGTGGGCAAGAAGTTCGAGAAGGACACCGGCATCAAGGTG
ACCGTCGAGCACCCAGATAAGCTGGAGGAGAAGTTCCCGCAGGTCGCCGCCACCGGCGACG
GCCCGGATATCATCTTCTGGGCCCACGACAGGTTCGGCGGCTACGCCCAGTCCGGCCTCCTC
GCCGAGATCACCCCGGACAAGGCCTTCCAGGATAAGCTCTACCCGTTCACCTGGGACGCCG
TGAGGTACAACGGCAAGCTCATTGCCTACCCGATTGCCGTCGAGGCCCTCTCCCTGATCTAC
AACAAGGACCTCCTCCCGAACCCGCCCAAGACCTGGGAGGAGATCCCGGCCCTCGACAAGG
AGCTGAAGGCCAAGGGCAAGTCCGCCCTCATGTTCAACCTCCAGGAGCCGTACTTCACCTG
GCCACTCATTGCGGCCGACGGGGGGTACGCCTTCAAGTACGAGAACGGCAAGTACGACATC
AAGGACGTGGGCGTGGACAACGCTGGCGCCAAGGCGGGCCTCACCTTCCTCGTGGATCTCA
TCAAGAACAAGCACATGAACGCCGACACCGACTACTCGATCGCCGAGGCCGCCTTCAACAA
GGGCGAGACCGCCATGACCATTAACGGCCCGTGGGCCTGGTCCAACATCGACACCTCTAAG
GTGAACTACGGCGTGACCGTGCTCCCGACCTTCAAGGGCCAGCCGTCCAAGCCGTTCGTGG
GCGTGCTCTCCGCCGGCATCAACGCCGCCTCCCCGAACAAGGAGCTGGCCAAGGAGTTCCT
GGAGAACTACCTCCTCACCGACGAGGGCCTGGAGGCCGTGAACAAGGACAAGCCGCTCGG
CGCCGTGGCCCTCAAGTCCTACGAGGAGGAGCTGGCGAAGGACCCGAGGATCGCCGCGACC
ATGGAGAACGCCCAGAAGGGCGAGATCATGCCGAACATCCCGCAGATGTCCGCCTTCTGGT
 
ACGCCGTCAGGACCGCCGTGATTAACGCCGCCAGCGGCAGGCAGACCGTGGACGAGGCCCT
GAAGGACGCCCAGACCAACTCTTCCTCGAACAATAACAATAACAACAATAATAACAACCTC
GGCGACGACGACGACAAGCTCCTCGGCGACTTCTTCCGCAAGTCCAAGGAGAAGATCGGCA
AGGAGTTCAAGAGGATCGTCCAGAGGATCAAGGACTTCCTCCGCAACCTCGTGCCAAGGAC
CGAGTCCAAGGATGAGCTGTGACTCGAGGAATTCGCGGCCGCACTCGAGATATCTAGACCC
AGCTTTCTTGTACAAAGTGGTGATGGGGGATCCACTAGTTCTAGAATTCGATTGAGTCAAGC
AGGATCGTTCAAACATTTGGCAATAAAGTTTCTTAAGATTGAATCCTGTTGCCGGTCTTGCG
ATGATTATCATATAATTTCTGTTGAATTACGTTAAGCATGTAATAATTAACATGTAATGCAT
GACGTTATTTATGAGATGGGTTTTTATGATTAGAGTCCCGCAATTATACATTTAATACGCGA
TAGAAAACAAAATATAGCGCGCAAACTAGGATAAATTATCGCGCGCGGTGTCATCTATGTT
ACTAGATCGACCGGCATGCAAGCTGATAT
**SEQ ID NO. 21:**
   Positions of individual functional components:
   B1 hordein promoter: 13 bp - 562 bp
   Kozak consensus sequence: 581 bp - 589 bp
   OsChtlsp: 590 bp - 646 bp
   6xHis: 647 bp - 664 bp
   Enterokinase-recognized restriction site: 665 bp - 679 bp
   LL-37: 680 bp - 790 bp
   Nos terminator: 818 bp - 1101 bp 
TTTAAAGTCGACGTGCAGGTGTATGAGTCATTGTTATGATCTATAGGTGTCAGTTTATCTTAT
CATCTGGGTGATCAATACAGGCCCAGGTTTTATAAAAACCAGTCGAGTCGAGAAGAACCGT
CCACATGTAAAGCTTTAACAACCCACACATTGATTGCAACTTAGTCCTACACAAGTTTTCCA
TTCTTGTTTCAGGCTAACAACCTATACAAGGTTCCAAAATCATGCAAAAGTGATGCTAGGTT
GATAATGTGTGACATGTAAAGTGAATAAGGTGAGTCATGCATACCAAACCTCGGGATTTCT
ATACTTTGTGTATGATCATATGCACAACTAAAAGGCAACTTTGATTATCAATTGAAAAGTAC
CGCTTGTAGCTTGTGCAACCTAACACAATGTCCAAAAATCCATTTGCAAAAGCATCCAAAC
ACAATTGTTAAAGCTGTTCAAACAAACAAAGAAGAGATGAAGCCTGGCTACTATAAATAGG
CAGGTAGTATAGAGATCTACACAAGCACAAGCATCAAAACCAAGAAACACTAGTTAACACC
AATCCACTGTCGACGGTACCGGATCCGCCACCATGAGGGCTCTCGCCGTGGTGGTGGTCGC
CACCGCCTTCGCTGTGGTGGCCGTGAGGGGCCACCATCACCATCATCATGACGACGACGAT
AAGCTCCTCGGCGACTTCTTCCGCAAGTCCAAGGAGAAGATCGGCAAGGAGTTCAAGAGGA
 
TCGTCCAGAGGATCAAGGACTTCCTGAGGAACCTCGTGCCGAGGACCGAGTCTTGACTCGA
GCCTTGGTCTAGAGAGCTCAGTCAAGCAGGATCGTTCAAACATTTGGCAATAAAGTTTCTTA
AGATTGAATCCTGTTGCCGGTCTTGCGATGATTATCATATAATTTCTGTTGAATTACGTTAAG
CATGTAATAATTAACATGTAATGCATGACGTTATTTATGAGATGGGTTTTTATGATTAGAGT
CCCGCAATTATACATTTAATACGCGATAGAAAACAAAATATAGCGCGCAAACTAGGATAAA
TTATCGCGCGCGGTGTCATCTATGTTACTAGATCGACCGGCATGCAAGCTGATATGAGCTCG
CATGCGCGGCCGCGATATC
**SEQ ID NO. 22,** not part of the invention:
   Positions of individual functional components:
   Oat globulin promoter: 1 bp - 960 bp
   Kozak consensus sequence: 989 bp - 997 bp
   8xHis: 998 bp - 1021 bp
   Enterokinase-recognized restriction site: 1022 bp - 1036 bp
   beta-defensin 2: 1037 bp - 1153 bp
   Nos terminator: 1169 bp - 1418 bp 
TGGAAAGTCATTTTGCCTCCTGAACTCCAGTGTTTCCTGTTTATTAAAAAAACTAAAAACTA
TACTTATAAGTTTGAAAAGATCATGAAACAAAATTGTAAAAATTGCTAATGATATATCCCAC
AAACGTGCAAAATCTCAATTCGAAGTGCTTTGTATTTCGAACTACACAAAAATGACAAAGT
GTGACTTTTTATGTGATTCGAAATCACTATACTACAGATCTACAATTTTGTCATTTTTGTGAA
GCTAAAAATACACATTATTTCGAATTGAGATTTTTCATGTTTGTGCTATGAATCACAGGCTA
CATCCTGATTTATTTTTAGAATTTTTTGGAACCCAAAATATGTTCTAGATTATTTTTTAAAAA
GTGGGATCACTTATGCCCATACACACGAAATCTCCACTCAATTCTTTTATACATTATCTTTCT
ATATCTACTAACGTGGATTATACATCATAGTAAGTTTCTTACTACATGTGCTTTCTTGTGACA
ATGTGGACACGACTCTTCCACTTTTGGGCTTTATGTTGTATTGATATACTCATGACATGGAAT
TTTGTCCACACACGTAGATCCATCCATATATATTGTTGTGCATAGAACGAAACACAAGCAAG
CCATTAAAAAGGAGTCACAAGTGCCACAAACTGTTGTAGGAAGTACAACTAGTATGAGGCC
TTTTATTTGACGTCGGACAATGGCCAAGAGCTACATACAAAAGATGGTGCTAGATTTGTGA
GTAAGCACCAGTTGTAGGCAGAAAACAACACATATCTTTTGAGGCAAAGTTATATCTATTC
ACATTTAAAACCATGATCTGTTGAGTCACCATGAATATCTTTTTATCTATGTTAATAATTACA
TGTCATCATGTTTATCCTGGACTACTTTTTATGGCTATAAAATCAAACTTACAATTAGGAAA
CTAGCACCAATCCACCTTCTACAATCTCGGATCCGTCCTAAAGCCGTACGGAATTCGCCACC
ATGCACCACCATCATCATCACCATCACGACGACGATGACAAGGACCCGGTGACCTGCCTCA
 
AGTCTGGCGCCATTTGCCACCCAGTGTTCTGCCCAAGGCGCTACAAGCAGATCGGCACGTG
CGGCCTCCCAGGCACCAAGTGCTGCAAGAAGCCAAACTGACTCGAGCCTAGGTCAAACATT
TGGCAATAAAGTTTCTTAAGATTGAATCCTGTTGCCGGTCTTGCGATGATTATCATATAATTT
CTGTTGAATTACGTTAAGCATGTAATAATTAACATGTAATGCATGACGTTATTTATGAGATG
GGTTTTTATGATTAGAGTCCCGCAATTATACATTTAATACGCGATAGAAAACAAAATATAGC
GCGCAAACTAGGATAAATTATCGCGCGCGGTGTCATCTATGTTACTAGATCGGG
**SEQ ID NO. 23,** not part of the invention:
   Positions of individual functional components:
   Trypsin inhibitor barley promoter: 1 bp - 2393 bp
   Kozak consensus sequence: 2406 bp - 2414 bp
   Pexiganan: 2415 bp - 2480 bp
   KDEL: 2481 bp - 2492 bp
   Nos terminator: 2508 bp - 2757 bp 
GTCGACGCATGTGTCATCTCATTCTGAAACGTGTGGTGCTGAGACGGTTGGAAATATGCCCT
AGAGGTAATGATAAATAGTTATTATATTTCTTGTTTCAAGATAATCATTTATTATTCATG
CTATAATTATATTGAATGAAAACATAGATACATGTGTGAATACATTGACGAAACAATGTCCT
TAGCAAGCCTCTAGTTGGCTAGCCAGTTGATCAAGGATAGTCAAGGTTTTCTGACTATGTGC
AAGTGTTGTCACTTGATAACTGGATCACATCATTAGGAGAATCATGTGATGGACTAGACCC
AAACTATGAATGTAGCATATTGATCGTGTCATTTTGTTGCTATTGTTTTCTGCGTGTCAAGTA
TTTATTCCTATGACCATGAGATCATATAACTCACTGGTACCGGAGGAATACCTTGTGTGTAT
CAAACGTCGCAACGTAACTGAGTGACTATAAAGGTGCTCTACAGGTATCTCTGAAGGTGTC
CGTTGAGTTAGTATGGATCAAGACTAGGATTTGCCACTCCGTGTGACGTAGAGGTATCTCGG
CCCACTCGGTAATACAACATCACACACAAGCCTTGCAAGCAATGTGACTAAGTTTAAGTCA
CGGGATCTTGTATTACCGAACGAGAAAAGAGACATGGATGCTAGTAGATTTCTTTTTGTAGA
GCACCTCACAACTAGACGGGCCCTGAAACACCTATACTTATATTTAGTATACAAAATGTTTG
TCCTGAAACAATACGTGAGTTGCAGAAAACGAAGCGACTTCGATAGACGCAGGAACACAC
GATGGAACGACCACGGAGCCGAAGGACGGCCGCGCGATCGGTCGGTACTAGATAATATTTT
CCCAAAAGATAATGCATGGATGCTAGTAGACTTATTTTCAGAAGTACTATACATACGATAC
GATACGACATATCATACCAAAATCGTATGCCAAATTATCGTATATAGAGTAATTCCGTTTCT
TTTTTGTCCCGGTGGATCTGAAATAACCACTTTCTGGAAAATCTTGGAAATTTAGGTTTTTTC
TGCCCGAATGAATTTAAAGGAACTTTGAATTTGATTTTTTTGCTGTGTGAAAATATATATAA
AGACGCTAACCTACTGACTAACGTAACTCCCTCATGCTCGACTGATCGGTTGGGTACACTCT
 
TTCGCCCGCCAGGTCATGAGATTGGATCCTCTCGATCGGTTGCAGACATGATCTCGACTCAT
AAACCAAACGAGAGTTTACTTGAAGTGCTAGATTACACTACAAATAAACATGGAAGAGAAT
GACATGCAGATGAACTAGGGATAATTCTGTTATCTCTTTTCGTAGGGTTCTTCTTGAACCTC
GACTGGCAGCATGGGATGAAGTACTCACTCGTTTGGAGGCTCGTTCAGCTGTCACATGGAT
GCTAGTAGATTTCTTTTCGTAGAGCCCCTCACAACCAGACGGGCCCCGAAACACCTATACTT
ATATCCAGTATAAAAAATGTCTGTCCTGAAACAATACGTGAGTTGCAGGAAAACGAAAGCG
GCTTCGATAGACGCAGGAACACACGAACGAACGGCCGCGCGATTGGTCGGTACTAGATAAT
ATTTTCCCAAAAGATAATGCATGGATGCTACTAGATTTTTTTCAGAAGTACTCTGCATACGA
TACGATATGACATATCATACAAAATCGTATGCCAAATTATCGTATTTAGAGTTAATTATGTT
TCTTTTTTGTTCGAGGGATCTGAAATAACCGGTTTCTGGAAAATCTTGTAAATTCAAGGTTTT
TCCGCCCGAATGAATTTAAACAAACTTTGAATTTGATTTTTTTGCAGTGTGAAAATATATAT
AAAGATGCTAACCTACTGACTAACGTAACTCCCTCATGCTCGACTGATCGGTTGCGTAGACC
CTGTCGCCCGCCAGGTCATGAGATTGGATCCTCTCGGCTGCAGACATGATCTCCACTCATAA
AGCAAACGAGAGTTTACTTGAAGTGTTAGATTACACATTTACACTACGAATAAACATGGAA
GAGAATGGCATGCAGATGAACCAGGGATGTAGCGTAGTATGATAAGATAACTCATGCCAAG
CACACTGAGAAAGGTAGGAACCGAGGAAATATGGTTATTCTTCTACTCAAACCCCAACTTTT
GCATTCCCCTCCGAATATTGTTAGCACAAAGCTCCAAATTCTGTCAGCAAAAAAGGTTATCA
AAGCTACCCTTTAATCAAAATTTCCATGCAGCGGCACTCCAACAACTAACAGAAAGTCAGA
AAGCACTTCGGTACTTACGATGCCAGCTGCTTGTCACTTCACATTTCACTATATATATACAT
GTGATACGCCTCGCTTGCTCCAACAACATCCCTTCAATTTGATAACACGTACGGAATTCGCC
ACCATGGGCATCGGCAAGTTCCTCAAGAAGGCCAAGAAGTTCGGCAAGGCCTTCGTGAAGA
TCCTGAAGAAGAAGGACGAGCTTTGACTCGAGCCTAGGTCAAACATTTGGCAATAAAGTTT
CTTAAGATTGAATCCTGTTGCCGGTCTTGCGATGATTATCATATAATTTCTGTTGAATTACGT
TAAGCATGTAATAATTAACATGTAATGCATGACGTTATTTATGAGATGGGTTTTTATGATTA
GAGTCCCGCAATTATACATTTAATACGCGATAGAAAACAAAATATAGCGCGCAAACTAGGA
TAAATTATCGCGCGCGGTGTCATCTATGTTACTAGATCGGG
**SEQ ID NO. 24 - LL-37 chimeric peptidic variant:**
   LLGDFFRKSKEKIGKEFKRIVQRIKDFLRNLVPRTESKDEL
**SEQ ID NO. 25 - LL-37 chimeric peptidic variant:**
   HHHHHHDDDDKLLGDFFRKSKEKIGKEFKRIVQRIKDFLRNLVPRTES
**SEQ ID NO. 26 - LL-37 chimeric peptidic variant:**

GGGGSGGGGSHHHHHHGGGGSGGGGSDDDDKLLGDFFRKSKEKIGKEFKRIVQRIKDFLRNLV
PRTES

The invention further includes a method of production of recombinant antimicrobial peptides selected from a group consisting of cathelicidins, defensins and magainins, comprising the following steps:
(a) providing a barley plant comprising in its genome a nucleotide sequence containing a sequence encoding an N-terminal signal peptide from a plant other than barley for translocation of a peptide through endoplasmatic reticulum, and a sequence encoding the antimicrobial peptide selected from a group consisting of cathelicidins, defensins and magainins, and optionally one or more sequences selected from the group comprising:
   - a sequence encoding a C-terminal sequence for retention of a peptide on endoplastmatic reticulum;
   - a sequence encoding at least one stabilizing and/or purification tag, such as a histidine tag or a maltose binding protein; a restriction site recognized by a protease such as enterokinase, DAMP4 biosurfactant protein, SUMO or SUMOstar peptide;
   preferably under the control of a promoter selected from the group comprising a grain-specific or constitutive promoter, wherein especially preferred promoters are ubiquitin promoter or a promoter controlling expression preferably in the caryopsis such as a B1 hordein promoter, a barley promoter of trypsin inhibitor, or an oat globuline promoter;
(b) cultivation of the barley plant until grains (caryopsises) are formed or, optionally, until the grains are ripe;
(c) isolation of the recombinant antimicrobial peptide or a chimeric variant thereof from the barley caryopsises (grains).

### Brief Description of Drawings

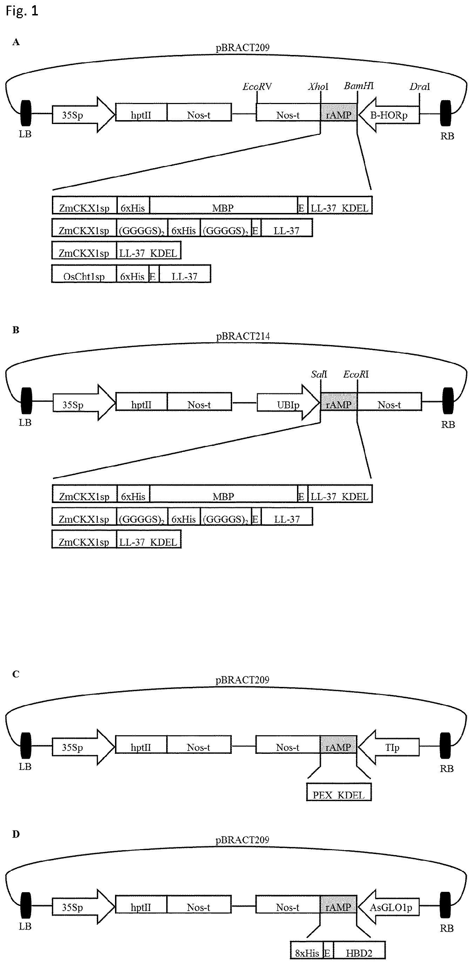

Fig. 1: Schematic representation of plant expression vectors used for genetic modification of barley. The coding sequences for individual recombinant antimicrobial peptides were inserted between the endosperm-specific B1-hordein promoter (A), the maize ubiquitin promoter (B), grain-specific trypsin inhibitor promoter (C), or the oat globulin promoter (D) and the nopaline synthase terminator (Nos terminator). A component of the final destination vectors was the *hptII* gene of resistance against hygromycin B, the expression of which was driven by the 35S promoter of cauliflower mosaic virus. rAMP: recombinant antimicrobial peptide; B-HORp: barley B1 hordein promoter; UBIp: maize ubiquitin promoter; Tip: promoter of barley trypsin inhibitor; AsGLO1p: oat globulin promoter; Nos-t: nopaline synthase terminator; ZmCKX1sp: signal peptide of maize cytokinin oxidase/dehydrogenase 1; OsCht1sp: signal peptide of rice chitinase 1; 6xHis / 8xHis: histidine tag; MBP: maltose-binding protein; E: restriction site recognized by enterokinase; LL-37: human antimicrobial peptide cathelicidin LL-37; PEX: antimicrobial peptide pexiganan, HBD2: human antimicrobial peptide beta-defensin 2; KDEL: tetrapeptide responsible for the retention in the endoplasmatic reticulum.

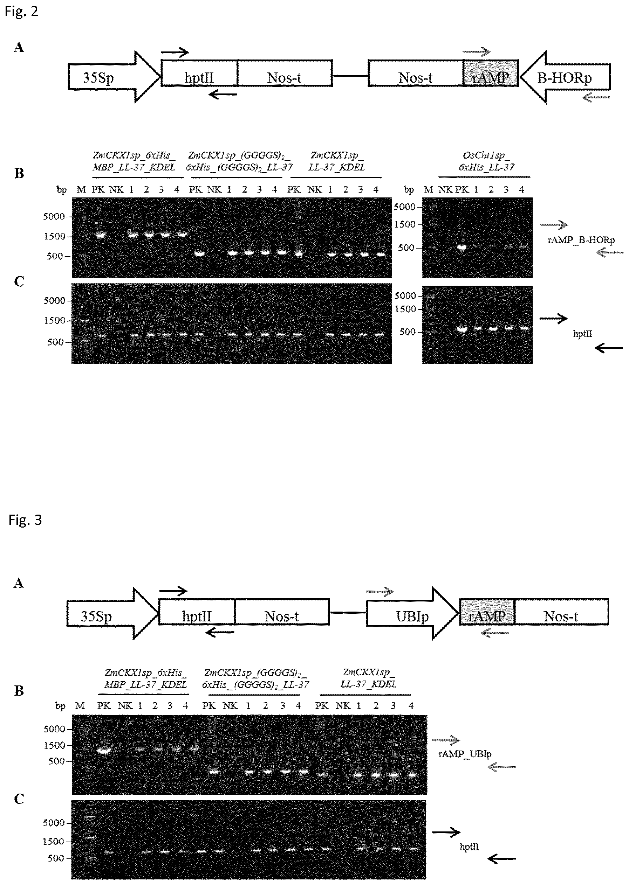

Fig. 2: The PCR detection of transgenes in genetically modified plants of T1 generation barley expressing the recombinant cathelicidin (rAMP) under the control of B1 hordein promoter (B-HORp). The detected area of transgenic DNA is indicated by arrows. The grey arrows correspond to the detection of *LL-37* and B1 hordein promoter, while the black arrows correspond to the *hptII* gene (A). Detection of *LL-37* gene and B1 hordein promoter. The amplicon sizes for the individual vectors used are 1681 bp (*ZmCKX1sp_6xHis_MBP_LL-37_KDEL*), 592 bp (*ZmCKX1sp_ (GGGGS)₂_6xHis_(GGGGS)₂_LL-37*)*,* 499 bp (*ZmCKX1sp_LL-37_KDEL),* and 538 bp (*OsCht1sp_6xHis_LL-37*) (B). Detection of the *hptII* gene. The amplicon size for all vectors used is 649 bp (C). M: molecular weight marker; PK: positive control (plasmid DNA carrying individual transgenes); NK: negative control (gDNA of control WT barley lines); 1, 2, 3, 4: independent lines with integrated relevant transgenes.
Fig. 3: The PCR detection of transgenes in genetically modified plants of T1 generation barley expressing the recombinant cathelicidin (rAMP) under the control of ubiquitin promoter (UBIp). The detected area of transgenic DNA is indicated by arrows. The grey arrows correspond to the detection of *LL-37* and ubiquitin promoter, while the black arrows correspond to the *hptII* gene (A). Detection of *LL-*37 gene and ubiquitin promoter. The amplicon sizes for the individual vectors used are 1379 bp (*ZmCKX1sp_6xHis_MBP_LL-37_KDEL),* 290 bp (*ZmCKX1sp_(GGGGS)₂*_*6xHis*_*(GGGGS)₂_LL-37*), and 197 bp (*ZmCKX1sp_LL-37_KDEL*) (B). Detection of *hptII* gene. The amplicon size for all vectors used is 649 bp (C). M: molecular weight marker; PK: positive control (plasmid DNA carrying individual transgenes); NK: negative control (gDNA of control WT barley lines); 1, 2, 3, 4: independent lines with integrated relevant transgenes.

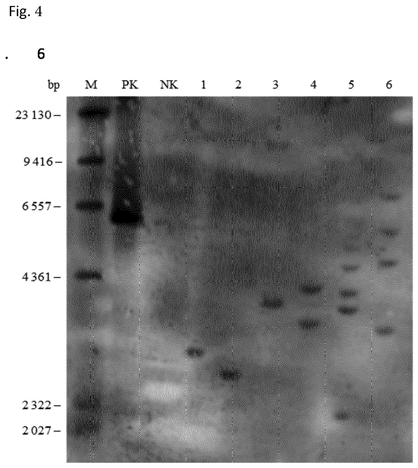

Fig. 4: Southern blot analysis of gDNA independent transgenic T0 lines of barley expressing the recombinant cathelicidin. gDNA (40 ug) was cleaved with the XhoI enzyme. The *hptII* gene was detected with the DNA probe. M: molecular weight marker; PK: positive control (plasmid DNA); NK: negative control (gDNA of control WT barley lines); 1, 2, 3, 4, 5, 6: independent transgenic barley lines with one (line 1 and 2), two (line 3 and 4) or more integrations (line 5 and 6).

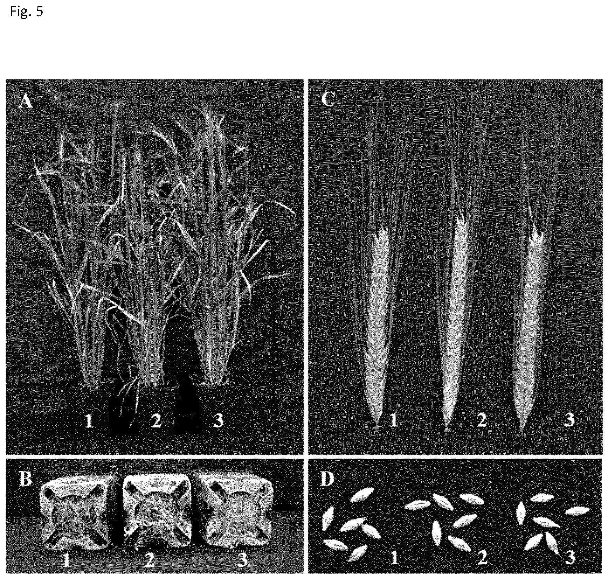

Fig. 5: Comparison of the phenotype of control WT barley plants (1) with the phenotype of T1 plants expressing the recombinant antimicrobial peptide under the grain-specific promoter (2) or maize ubiquitin promoter (3). Phenotype of the above-ground part of barley plants (A). Phenotype of the root system (B). Phenotype of spikes (C). Phenotype of ripe grains (D).

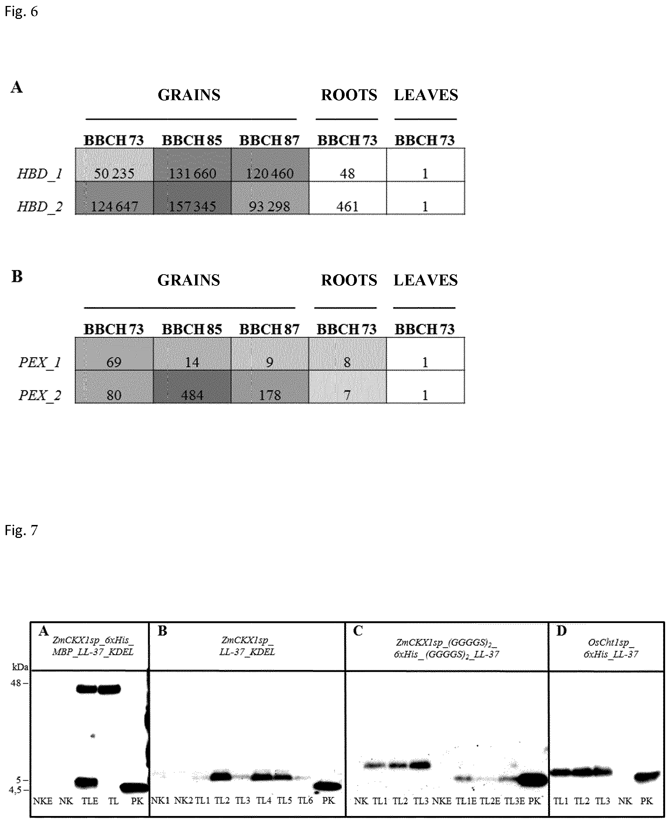

Fig. 6: Relative quantification of expression of the human antimicrobial peptide beta-defensin 2 (*HBD2,* A) derivative or antimicrobial peptide pexiganan (*PEX,* B) in different transgenic barley tissues in plants transformed by construct AsGLO1p::8xHis_HBD2_KDEL::Nos (A) or TIp::PEX_KDEL::Nos (B) relative to the basal expression detected in the leaves. The expression was relatively quantified always in 4 to 6 different biological replicates for each independent transgenic line and the analysis was always set in three technical replicates per each biological replicate. The DataAssist^{™} software was used to evaluate data using the delta delta Ct method. *HBD2_1* and *HBD2*_*2*: independent transgenic line of T2 generation barley expressing *HBD2;PEX_1* and *PEX_2:* independent transgenic line of T1 generation barley expressing *PEX.* 73, 85, 88: three different stages of development of barley determined by the consistency of spike used for the analysis (classification by the BBCH scale).
Fig. 7: Western blot detection of recombinant cathelicidin in protein extracts prepared from the milky-ripe grains of transgenic barley lines expressing the represented chimeric *LL-37* genes (A), (B), (C), (D) under the control of B1 hordein promoter. NK: negative control (protein extract from the control WT barley lines); NKE: negative control cleaved with enterokinase; TL: protein extract from the transgenic line; TLE: cleaved protein extract from the transgenic line; PK: positive control (synthetic antimicrobial peptide LL-37, 10 ng). Theoretical size of recombinant cathelicidin for individual chimeric variants: 48 kDa and 5 kDa respectively for the cleaved product (A); 5 kDa (B); 7.2 kDa and 4.5 kDa respectively for the cleaved product (C); 5.9 kDa (D). Size of the synthetic cathelicidin LL-37: 4.5 kDa.

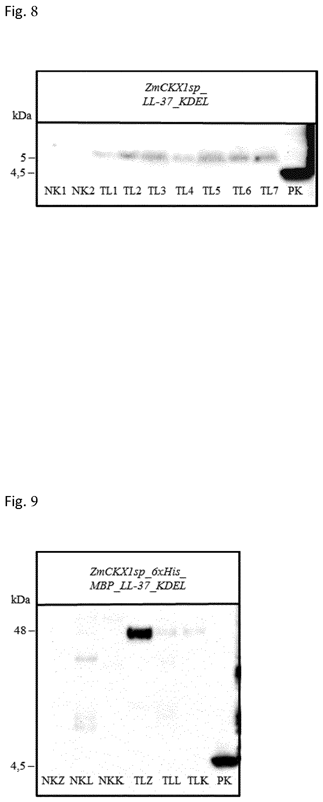

Fig. 8: Western blot detection of recombinant cathelicidin in protein extracts prepared from the ripe grains of transgenic barley lines expressing the shown chimeric *LL-37* gene under the control of B1 hordein promoter. NK: negative control (protein extract from the control WT barley lines); TL: protein extract from the transgenic line; PK: positive control (synthetic antimicrobial peptide LL-37, 10 ng). Theoretical size of recombinant cathelicidin for the given chimeric variant: 5 kDa. Size of the synthetic cathelicidin LL-37: 4,5 kDa.
Fig. 9: Western blot detection of the recombinant cathelicidin in protein extracts prepared from different tissues of transgenic barley lines expressing the shown chimeric *LL-37* gene under the control of maize ubiquitin promoter. NK: negative control (protein extract from the grains, leaves or roots / NKZ, NKL or NKK of the control WT barley lines); TL: protein extract from the grains, leaves or roots / TLZ, TLL or TLK transgenic lines; PK: positive control (synthetic antimicrobial peptide LL-37, 10 ng). Theoretical size of recombinant cathelicidin for the given chimeric variant: 48 kDa. Size of the synthetic cathelicidin LL-37: 4,5 kDa.

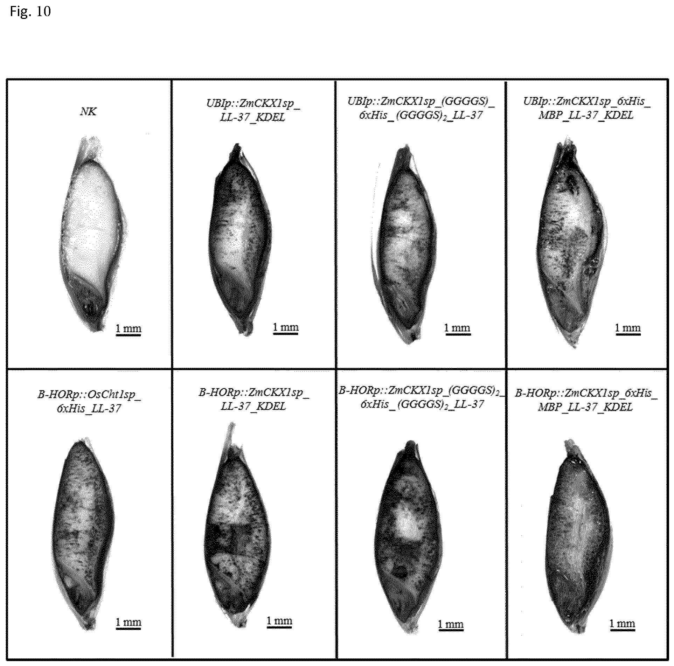

Fig. 10: Immunodetection of recombinant cathelicidin in ripe grains of the T1 generation barley, using an antibody specific against LL-37. NK: negative control (grain of the control WT line).

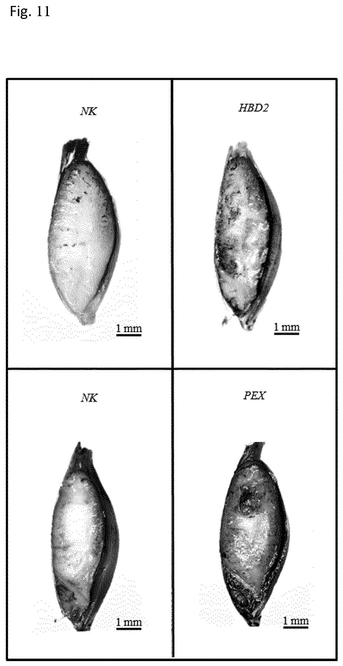

Fig. 11: Immunodetection of recombinant defensin (HBD2) and pexiganan (PEX) in ripe grains of barley, using antibodies specific against individual antimicrobial peptides. NK: negative control (a grain of the control WT line).

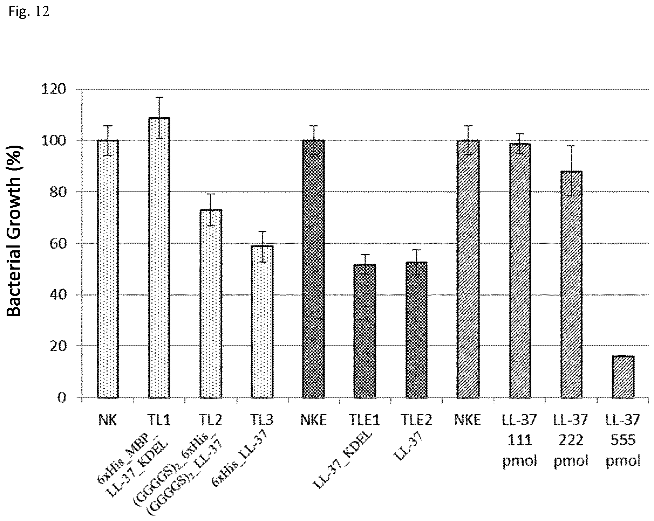

Fig. 12: *In vitro* antimicrobial activity of the recombinant LL-37 products against TOP10 *Escherichia Coli* bacteria Purified protein extracts were prepared from milky-ripe grains of the T1 barley line. The tested recombinant LL-37 products for individual lines are represented in the graph. Growth of the bacterial population, in the presence of control extracts (10 µg extracted proteins) from the grains of the control WT lines (negative control; NK) or in the presence of extracts (10 µg) from the control lines treated with enterokinase (NKE) was considered to be 100 percent. TL: extract from the grains of transgenic lines (10 µg) with an integrated gene: *ZmCKX1sp_6xHis_MBP_LL-37_KDEL* (TL1), *ZmCKX1sp_(GGGGS)₂*_*6xHis_(GGGGS)₂_LL-37* (TL2), *OsCht1sp_6xHis_LL-37* (TL3). TLE: the enterokinase-treated extract from the grains of transgenic lines (10 µg) with an integrated gene: *ZmCKX1sp_6xHis_MBP_LL-37_KDEL* (TLE1), *ZmCKX1sp_(GGGGS)_{2_}6xHis*_ *(GGGGS)2_LL-37* (TLE2). The synthetic peptide LL-37 applied to the purified extracts from the control WT lines in the indicated amount served as a positive control. Error line segments represent a mean average error (SE).

### Examples

Where the examples refer to constructs and sequences containing N-terminal signal peptide other than N-terminal transient signal peptide of maize cytokinin oxidase/dehydrogenase 1 or N-terminal signal peptide of rice chitinase 1, such constructs and sequences do not form part of the claimed invention.

### Example 1: Preparation of a genetically modified barley

### 1. Preparing an expression cassette for introducing foreign DNA into barley

Seven expression vectors were prepared, containing different chimeric variants of *LL-37* gene under the control of the barley grain-specific B1 hordein promoter (Fig. 1A) or the maize ubiquitin promoter (Fig. 1B). The sequence of the promoter for B1 hordein barley protein (GenBank: X87232.1) has been synthesized to order by Mr. Gene GmbH (Regensburg, Germany) together with the Nos terminator sequence. A multiple cloning site with unique restriction sites recognized by specific endonucleases was placed between the sequence for B1 hordein promoter and the Nos terminator to introduce the corresponding chimeric variants of *LL-37* gene (SEQ ID NO. 1; SEQ ID NO. 2; SEQ ID NO. 3; SEQ ID NO. 4). Individual *LL-37* variants have been codon-optimized for barley and synthesized to order by Thermo Fisher Scientific (Walthan, MA, USA). The synthesized *LL-37* variants included also the initiating Kozak consensus sequence, which was designed taking into account the preference of the individual nucleotides occurring around the initiation codon in barley. Restriction sites for subsequent introduction of the respective sequences into the input vectors were placed at the beginning and the end of each *LL-37* gene. Other DNA sections introduced into barley were obtained by purchasing the input pENTR^{™} 2B Dual Selection Vector (Thermo Fisher Scientific) or pENTR^{™} 1A Dual Selection Vector (Thermo Fisher Scientific) and by purchasing the binary T-DNA plasmid pBRACT209 or pBRACT214 (John Innes Centre, Norwich, Great Britain).

For the grain-specific expression of variants of *LL-37* gene controlled by the barley B1 hordein promoter, the promoter sequence was first introduced together with the Nos terminator sequence by restriction endonucleases *Dra*I and *EcoR*V (New England Biolabs, **I**pswich, MA, USA) and T4 DNA ligase (New England Biolabs) into the input pENTR^{™} 1A vector. Subsequently, the individual chimeric *LL-37* genes were cloned between the promoter and the terminator using the *BamH*I and *Xho*I endonucleases and T4 DNA ligase. Then, the individual functional segments were introduced into the target vector pBRACT209 using Gateway cloning. For this purpose Gateway^{™} LR Clonase^{™} II Enzyme mix (Thermo Fisher Scientific) was used. Cloning was performed according to the manufacturer's recommended instructions. For the expression of the variants of *LL-37* gene controlled by the maize ubiquitin promoter, which was part of the commercial pBRACT214 vector like the Nos terminator, the individual genes were first introduced by restriction endonucleases *Sal*I and *EcoR*I and T4 DNA ligase into the input pENTR^{™} 2B Dual Selection Vector. It served as a donor vector for introducing the desired DNA section into the target vector pBRACT214 using Gateway cloning with the Gateway^{™} LR Clonase^{™} II Enzyme mix.

Furthermore, one expression vector containing the chimeric variant of an antimicrobial peptide pexiganan (Fig. 1C) and one vector with a gene encoding a human beta-defensin 2 derivative (Fig. 1D) were prepared. For the grain-specific expression of pexiganan, the barley trypsin inhibitor promoter sequence (GenBank: X65875.1) was used; the HBD2 expression was controlled by the promoter for the globulin storage protein of oat. The variants were codon- optimized for barley codon and, together with the Nos terminator, synthesized by Thermo Fisher Scientific. At the beginning and end of each sequence, the attl1 or attl2 sites were placed for enabling the Gateway cloning, which was done in the same way as in the case of preparation of the transformation cassettes (expression cassettes) with chimeric *LL-37* genes. The pBRACT209 was used as the target vector.

The resulting constructs were verified by restriction analysis, PCR reaction and commercial sequencing (SEQme, Dobříš, Czech Republic) and subsequently introduced together with the pSOUP helper plasmid by electroporation into the bacterium *Agrobacterium tumefaciens* (AGL1 strain), which was obtained from the Plant Breeding and Acclimatization Institute (Blonie, Poland).

### 2. Transforming barley by means of Agrobacteria tumefaciens

Transformation of barley by means of *Agrobacteria tumefaciens* proceeded according to the published protocol (Harwood, W. A. et al. in Transgenic Wheat, Barley and Oats: Production and Characterization Protocols; 137-147; Humana Press, New York, USA, 2009). The protocol was modified by adding 0.3 mM acetosyringon to the agrobacterial suspension prior to the infection of the donor material, which were the immature embryos of Golden Promise spring barley cultivar (barley seeds obtained from the John Innes Centre, Norwich, Great Britain). Another modification was the addition of biotin at a final concentration of 0.1 mg·l⁻¹ to the bacterial MG/L culture medium.

### 3. Growing transgenic barley

*In vitro* cultivation and plant regeneration proceeded according to the published protocol (Harwood, W. A. et al. in Transgenic Wheat, Barley and Oats: Production and Characterization Protocols; 137-147; Humana Press, New York, USA, 2009). After the plants were transplanted into peat pellets, the plants were grown in a phytotron (16 hours day - 15 °C, 8 hours night - 12 °C; relative humidity 60 %; light intensity 140 µM·m⁻²·s⁻¹ (at the level of growing pots). Finally, the plants were transplanted into a mixture of perlite and a commercial Gramoflor substrate, special mix (Gramoflor GmbH & Co., Vechta, Germany) in a ratio of 10 g of perlite/1 litre of substrate.

As a control for the necessary analyses, plants were prepared which have undergone a regeneration process from tissue cultures but have not been infected with the *Agrobacterium* suspension. The media for the cultivation of control plants did not contain antibiotics.

From each of the expression vectors used (Fig. 1), at least 5 (at most 37) independent T0 transgenic lines were obtained. The efficiency of the transformation was approximately 10 percent. The hygromycin resistance gene (hpt II), which was part of both pBRACT vektors used, served as a marker for transgenic plant selection.

### 4. Selecting diploid lines

Since ploidy changes can occur due to regeneration of plants from tissue cultures, the number of homologous sets of chromosomes was determined in the T0 transgenic lines using the flow cytometry. To a young leaf segment (approximately 1 cm²) from the transgenic and control WT barley was added 1.5 ml buffer (15 mmol·l⁻¹ Tris, 2 mmol·l⁻¹ EDTA disodium salt, 0.5 mmol·l⁻¹ spermine, 80 mmol·l⁻¹ potassium chloride, 20 mmol·l⁻¹ sodium chloride, 0.1 % Triton X-100, 15 mmol·l⁻¹ β-mercaptoethanol, pH 7.8), in which the leaves were shredded into small pieces by means of a razor blade. After filtering the extract, 50 µl 1mg·ml⁻¹ ribonucleases and 50 µl 0.1 mg·ml⁻¹ DAPI fluorescent dye was added to the suspension of the released nuclei. The plant ploidy level was determined by CyFlow^{®} Space analyser (Sysmex Partec GmbH, Görlitz, Germany). Only diploid barley lines were selected for subsequent analyses.

### 5. Detecting transgene using PCR analysis and Southern blot method

Genomic DNA was isolated from the leaves of approximately 4-month old T0 and T1 barley plants. The transgene integration was verified at the genomic level by the conventional PCR method using always at least two sets of gene-specific primers. All the lines were verified for the presence of *hptII* gene (5'-CGAAAAGTTCGACAGCGTC-3' and 5'-GGTGTCGTCCATCACAGTTTG-3'), which was part of the commercial pBRACT vectors (Fig. 2, Fig. 3). Furthermore, depending on the nature of the construct used, the presence of B1 hordein promoter (B-HORp) together with *LL-37* gene (5'-TCCATTCTTGTTTCAGGCTAAC-3' and 5'-GCCGATCTTCTCCTTGGACTT-3'; Fig. 2), ubiquitin promoter (UBIp) together with *LL-37* gene (5'-TGCTCACCCTGTTGTTTGGTGTTAC-3' and 5'-GCCGATCTTCTCCTTGGACTT-3'; Fig. 3) was detected. The gDNA from leaves of control WT plants was used as a negative control, while the plasmid DNA with individual genes was used as a positive control.

For the transgenic T0 lines selected as positive by the PCR analysis the number of copies of the insert were determined by Southern blot method using the DNA probe complementary to *hpt II* gene. 40 µg of genomic DNA was cleaved with XhoI enzyme (New England Biolab), the fragments obtained were mixed with the loading buffer, DNA Gel Loading Dye (Thermo Fisher Scientific), separated by gel electrophoresis (0.8 % agarose) and transferred to the nylon membrane BLOTTING-Nylon 66 Membranes, Type b, positive for Northern/Southern blotting (Merck, Kenilworth, NJ, USA). The DNA Molecular Weight Marker Digoxigenin-labelled 0.12-23.1 (Merck) was used as a molecular weight standard. Subsequently, the digoxygenin-labelled DNA probe specific for *hptII* gene was synthesized using gene-specific primers 5'-GAATTCAGCGAGAGCCTGAC-3' and 5'-ACATTGTTGGAGCCGAAATC-3'. A blank pBRACT207 plasmid served as a template. The probe was synthetised using the DIG Probe Synthesis Kit (Roche, Basel, Switzerland). The DIG-EasyHyb (Roche) commercial kit was used to hybridize immobilized fragments. To detect gDNA fragments with a bound probe, anti-Digoxigenin-AP Fab fragments antibody (Roche) was used, followed by a CSPD chemiluminescent substrate solution (3-(4-metoxyspiro{1,2-dioxetan-3,2'-(5'-chloro)tricyclo[3.3.1.13,7]decan}-4-yl)phenyl sodium phosphate, Roche).

It was found that most lines contained more than one copy of the corresponding integrated T-DNA (Fig. 4). Neither the constitutive nor the grain-specific expression of the antimicrobial peptide had any negative effect on the phenotype, rate of development or fertility of the modified lines of barley, regardless of the number of transgene integrations (Fig. 5).

In order to derive the T1 generation, the lines that were positive for the detected transgenes using both sets of gene-specific primers (PCR analysis) and simultaneously also by means of the Southern blot method were selected. In the T1 generation, the structural and functional stability of the individual transgenes was subsequently monitored.

### Example 2: Study of transgenic DNA expression

### 1. RNA-level analysis

In GMO barley lines expressing the human beta-defensin 2 derivative (HBD2) or the antimicrobial peptide *Xenopus laevis* magainin 2 derivative pexiganan (PEX), the number of transgene transcripts in roots, leaves, or grains was relatively quantitated in three different growth stages classified according to the Macrophenological scale for cereals BBCH as 73, 85 and 87 (early milk, soft dough and hard dough, respectively). RNA was isolated using the RNAqueous^{®} kit (Thermo Fisher Scientific) and after being treated with Turbo DNase (Thermo Fisher Scientific), it was purified with magnetic beads (Agencourt RNA-CLEAN XP, Beckman Coulter, Brea, CA, USA). This was followed by the transcription into cDNA by reverse transcriptase RevertAid H Minus M-MULV (Thermo Fisher Scientific).

The expression of *HBD2* or *PEX* transgenes was relatively quantified by SYBR^{®} Green (in the case of *HBD2)* or TaqMan^{®} (in the case of *PEX*) chemistry using the real-time PCR system (ViiA 7, Thermo Fisher Scientic). Gene-specific primers were designed using Primer Express 3.0 program. Genes for barley β-actin (Act), barley cyclophilin (CYCLO) and elongation factor 2 (EF2) served as an endogenous control. Table 1 summarizes the list of primers used.

In all analysed lines the transgenic RNA was detected in grains at all stages of growth, with the average relative amount of transgene transcripts in the tissue being always significantly higher relative to the amount of transgenic RNA in the leaves or roots (Fig. 6). Moreover, Figure 6 shows that the expression of transgene controlled by the barley endogenous promoter for the trypsin inhibitor protein is also slightly increased in the roots, whereas the oats globulin promoter appears to be significantly grain-specific and only very weak expression at the level of noise was detected in the leaves.

**Table 1 Sequences of primers used for qRT-PCR analysis**

| **Name** | **Target gene** | **Forward primer sequence (5'→3')** | **Reverse primer sequence (5'→3')** | **Probe sequence (5'→3')** | **Chemist ry** |
|---|---|---|---|---|---|
| EF2 | Elongation factor 2 | AAGTCCTGCCGCACTGTC AT | GGGCGAGCTTCCATGT AAAG | AGCAAGTCCCCCAACAA GCATAACCG | TaqMan |
| ACT | Actin | TGTTGACCTCCAAAGGAA GCTATT | GGTGCAAGACCTGCTG TTGA | TGTAGTATTCAGCTGGTT GGTGGCACAGC | TaqMan |
| CYCLO | Cyclophilin | TGTCTATGGATTTGACAC CACTCTTT | GAAGCCTGCCCGAAGC A | TGACCTGTTTCTTCGCA CACCAGCC | TaqMan |
| HBD2 | Defensin | AAGTCTGGCGCCATTTGC | CACGTGCCGATCTGCT TGTA | ACCCAGTGTTCTGCCCAA GGCG | TaqMan |
| EF2 | Elongation factor 2 | CCGCACTGTCATGAGCAA GT | GGGCGAGCTTCCATGT AAAG | - | SYBR Green |
| ACT | Actin | TTGACCTCCAAAGGAAGC TATTCT | GGTGCAAGACCTGCTG TTGA | - | SYBR Green |
| HvCYC LO | Cyclophilin | CCCAGTTCTACATAACCA CAATCAA | ACCCTGCCAAAGACTA CATGCT | - | SYBR Green |
| PEX | Pexiganane | AAGTTCCTCAAGAAGGCC AAGAA | CTTCTTCAGGATCTTCA CGAAGG | - | SYBR Green |

### 2. Protein-level analysis

### 2.1 Purification of recombinant LL-37 products

After the barley roots, leaves or milky-ripe grains were homogenized, the extraction buffer of 0.1 mol·l⁻¹ Tris-HCl, 0.3 mol·l⁻¹ sodium chloride, 0.01 mol·l⁻¹ imidazol, 4 % (v/v) glycerol, 0.3 % (w/v) Triton X-100, EDTA-free protease inhibitor cocktail (Roche), pH 8.0 was added and samples were incubated for 2 hours at 4 °C. After centrifugation at 12,000 g / 30 min / 4 °C, the supernatant was removed and the pellet was subsequently re-extracted in the same manner. Then the combined extracts were purified by the immobilized metal-chelate affinity chromatography (IMAC) using the chromatographic columns packed with Co²⁺-IDA-agarose (Qiagen, Germantown, MR, USA). The bound proteins were eluted with 400 mM imidazole, whereas neither washing nor elution buffer contained Triton X-100. Finally, the extracts were washed 1: 5 (v/v) with 5 mM NH₄HCO₃ (pH 8.0) and concentrated to the desired final volume by means of the Amicon membrane filters.

### 2.2 Proteolytic cleavage of fusion tags

The chimeric genes *ZmCKX1sp_6xHis_MBP_LL-37_KDEL* and *ZmCKX1sp*_*(GGGGS)₂_6xHis_ (GGGGS)_{2_}LL-37* contained a sequence encoding a restriction site recognized by enterokinase. In the case of *ZmCKX1sp*_*6xHis_MBP_LL-37_KDEL* gene, the said sequence was located between the encoding sequence for MBP and LL-37, and one of the cleavage products was therefore LL-37_KDEL (Fig. 7A), while in the case of *ZmCKX1sp_(GGGGS)₂_6xHis*_*(GGGGS)₂_LL-37* gene, the sequence was behind the flexible linking sequence (GGGGS)₂ and LL-37, and one of the cleavage products was LL-37 (Fig. 7C). Enterokinase (New England Biolabs) was used for proteolytic cleavage of fusion tags from the recombinant peptide LL-37. Purified protein samples were washed 1:5 (v/v) with buffer for enterokinase (20 mmol·l⁻¹ Tris-HCl, 50 mmol·l⁻¹ sodium chloride, 2 mmol·l⁻¹ calcium chloride, pH 8.0) and then the enzyme 1:30 (v/v) was added. After proteolytic cleavage at 23 °C for 16 h, the samples were washed 1:5 (v/v) using 5mM NH₄HCO₃ (pH 8.0) and concentrated to the desired final volume by means of the Amicon membrane filters. The cleavage efficiency was verified by the Western blotting method based on the detection of newly created signals for the respective peptides which were not present in the corresponding uncleaved fractions and whose electrophoretic mobility was the same as the theoretical size of the LL-37 cleavage products (Fig. 7A, Fig. 7C).

### 2.3 Western blot detection of recombinant cathelicidin

For the Western blot detection (Fig. 7; Fig. 8; Fig. 9), both cleaved and uncleaved purified protein extracts (400 ug) were precipitated with 85 % acetone. After being dissolved in a mixture of water, a loading buffer (Thermo Fisher Scientific) and a reducing agent (Thermo Fisher Scientific), they were heated to 70 °C for 10 minutes and then electrophoretically separated using a commercially available SDS-PAGE gel Bolt 4 - 12% Bis-Tris Plus (Thermo Fisher Scientific). After transfer to the PVDF Immunobilion^{®} - P Transfer Membrane (Merck), the immunoreaction was performed using a commercially available polyclonal primary antibody against LL-37 (Santa Cruz Biotechnology, Dallas, TX, USA) and a secondary antibody Goat anti-rabbit (Santa Cruz Biotechnology). The primary antibody was diluted at 1:400 and the secondary antibody was diluted at 1:1000. A semi-automatic iBind^{™} Western system (Thermo Fisher Scientific) was used to block the PVDF membrane and deposition of antibodies including washing steps. The amount of the recombinant LL-37 peptide was quantified by Image Lab^{™} Software, wherein a five-point calibration curve of the signal intensity dependence on the LL-37 synthetic peptide concentration was first assembled and then the amount of LL-37 for each line was determined.

The Western blot method demonstrated the functionality of the expression vectors used. Independently of the character of the construct used, it was possible to detect the recombinant LL-37 in grains of transgenic lines (Fig. 7; Fig. 8; Fig. 9), wherein the line with grain-specific LL-37 expression accumulated a larger amount of antimicrobial peptide in caryopses (grains) than lines in which the expression was controlled by maize ubiquitin promoter, which is considered to be a moderate promoter for use in cereals. For this reason, the B1 hordein promoter appears to be a suitable promoter for expression of a target gene in the grains of monocotyledonous plants for the molecular farming purposes (Tab. 2).

**Table 2: Approximate amount of LL-37 antimicrobial peptide in milky-ripe transgenic caryopses of T1 generation barley; B 1-HORp: expression controlled by barley B1 hordein promoter; UBIp: expression controlled by maize ubiquitin promoter; N. D.: not detected.**

| Chimeric LL-37 gene | Amount of LL-37 (pmol) in 1 grain | |
|---|---|---|
| | B1-HORp | UBIp |
| *ZmCKX1sp_(GGGGS)₂_6xHis_(GGGGS)₂_LL-37* | 1.533 | N. D. |
| *ZmCKX1sp_LL-37_KDEL* | 4.000 | 0.100 |
| *ZmCKX1sp_6xHis_MBP_LL-37_KDEL* | 0.011 | 0.006 |
| *OsCht1sp_His_LL-37* | 0.688 | Lines not prepared |

The LL-37 was quantified by Image Lab^{™} Software, which determined the dependence of signal intensity on the LL-37 synthetic peptide concentration. The approximate amount of recombinant peptide LL-37 was subsequently determined by means of an assembled calibration curve and converted to the equivalent amount of LL-37 based on the known theoretical molecular weight values of the respective recombinant variants of peptide LL-37 and the LL-37.

In the lines expressing the recombinant cathelicidin under the control of the constitutive ubiquitin promoter, it was possible to detect the chimeric LL-37 variant also in the roots or leaves (Fig. 9).

### 2.4 In situ immunodetection of recombinant cathelicidin in ripe barley grains

For the purpose of a more detailed characterization of the individual lines, immunolocalization of the recombinant cathelicidin was performed in the ripe T1 generation grains according to the published protocol (Qu LQ, Tada Y, Takaiwa F. (2003) Plant Cell Rep. 22, 282-285). Modifications consisted of prolonged blocking (4 hours) and use of 5% bovine serum albumin (BSA) in TBS buffer (w/v), with subsequent incubation with the primary antibody against LL-37 (Santa Cruz Biotechnology) at 1:40 (v/v) dilution in TBS buffer containing 2.5% (w/v) BSA. The secondary antibody was Goat anti-rabbit (Merck) diluted at 1:2 500 (v/v) in TBS buffer containing 2.5% (w/v) BSA. Incubation with secondary antibody was carried out for 1.5 h at 37 °C and then for 1.5 h at room temperature. The colorimetric detection of LL-37 was performed using an NBT/BCIP (nitroblue tetrazolium/5-bromo-4-chloro-3-indolylphosphate) substrate for alkaline phosphatase (Merck) incubated with the samples for 30 minutes. The reaction was stopped by replacing the substrate with sterile water. A stereo microscope Nikon SZM800 was used to capture images.

The positive reaction to the presence of peptide LL-37 was manifested by endosperm staining in all analysed lines (Fig. 10). In addition to the purple colour of the endosperm, the staining of embryos in individual lines could also be observed, but it could not be attributed to the specific detection of LL-37, because a similar signal was also yielded by the grains of the control WT barley lines.

### 2.6 In situ immunodetection of a variant of the antimicrobial peptide pexiganan and the human beta-defensin 2 derivative in ripe barley grains

By immunolocalization, the presence of the derivative of the antimicrobial peptide pexiganan (PEX) and the human beta-defensin 2 (HBD2) in the ripe barley caryopsis was further demonstrated. From a methodological point of view, immunolocalization was performed in the same way as in the case of detection of recombinant LL-37 products (see point 5). For PEX detection, a rabbit polyclonal primary antibody was used that was synthesized to order by EXBIO Praha (Vestec, Czech Republic) and Goat anti-rabbit (Merck) served as the secondary antibody. For HBD2 detection, the primary antibody was a monoclonal antibody against HBD2 of murine origin (Abeam, Cambridge, Great Britain), with Goat anti-mouse (Merck) as the second antibody. The dilutions of primary and secondary antibodies were the same as for the LL-37 detection.

The positive reaction showing the presence of HBD2 and PEX was manifested by endosperm staining in all analysed lines (Fig. 11). In the case of pexiganan detection, anonspecific staining of embryos of the individual lines (as well as in LL-37 detection) could be observed, while in the detection of HBD2 the signal was observed only in the endosperm. The differences can be attributed to the different specificity of the antibodies used.

### Example 3: In vitro testing of antimicrobial activity of recombinant peptides

The antimicrobial activity of recombinant LL-37 products was tested against *E. coli* strain TOP 10. First, the bacterial culture was freshly grown in antibiotic-free liquid Luria-Bertani (LB) medium. From the culture in the exponential growth phase, bacteria were removed under aseptic conditions and added to 10 µl purified protein samples in 5 mmol.l⁻¹ NH₄HCO₃ (the number of proteins in each sample was approximately 10 µg). The final concentration of bacteria corresponded to a density of 1×10⁷ CFU/ml ("a colony forming unit" per millilitre). The samples were incubated for 4 h in sterile microtips at 37 °C with constant stirring (1,000 rpm), evenly spread on antibiotic-free LB medium with agar, and then incubated for a period of 24 h at 37 °C. The number of colonies was then determined on the individual dishes and the result was used to calculate CFU/ml. Testing of antimicrobial activity was performed in at least two independent experiments, where always fresh protein extracts were prepared. Each biological sample was set in 3 technical replicates. The synthetic peptide LL-37 (LifeTein LLC, Hillsborough, NJ, USA) applied at different concentrations to the purified extract from control non-transgenic lines was used as a positive control.

It has been found that the heterologously produced peptide LL-37 without additional sequences exhibits antimicrobial properties (Fig. 12) with approximately the same potency as its peptide variant with the bound C-terminal KDEL sequence (SEQ ID NO. 24). Furthermore, it has been found that antibacterial activity of the peptide is also maintained when a histidine tag is attached to the N-terminus of cathelicidin LL-37, either alone (SEQ ID NO. 25) or bounded in both sides by the linker sequence GGGGS (SEQ ID NO. 26), and therefore it is not necessary for the biological activity to be cleaved away by means of expensive protease.

## Claims

1. A method of production of a barley plant producing at least one antimicrobial peptide, comprising the step of insertion by transformation of a nucleotide sequence containing
- a sequence encoding an N-terminal signal peptide from a plant other than barley for translocation of a peptide through endoplasmatic reticulum, selected from N-terminal transient signal peptide of maize cytokinin oxidase/dehydrogenase 1 and N-terminal signal peptide of rice chitinase 1, and
- a sequence encoding at least one antimicrobial peptide selected from a group consisting of cathelicidins, defensins and magainins,
into the genome of the barley plant;
and wherein the nucleotide sequence is under the control of a grain-specific or a constitutive promoter selected from a ubiquitin promoter, a B1 hordein promoter, a trypsin inhibitor barley promoter, an oat globulin promoter.

2. The method according to claim 1, **characterized in that** the nucleotide sequence further contains one or more sequences selected from the group consisting of
- a sequence encoding a C-terminal sequence KDEL for the retention of a peptide on endoplasmatic reticulum;
- a sequence encoding at least one stabilization and/or purification tag selected from a histidine tag, a maltose-binding protein, a restriction site recognized by a protease, histidine tag, SUMOstar, putative barley SUMO sequence, DAMP4 biosurfactant protein and/or flexible binding sequence (GGGGS)₂.

3. The method according to claim 1 or 2, wherein the antimicrobial peptide is LL-37.

4. The method according to any one of the preceding claims, wherein the grain-specific promoter is the B1 hordein promoter.

5. The method according to claim 2, wherein the stabilization and/or purification tag is the histidine tag and/or flexible binding sequence (GGGGS)₂.

6. The method according to any one of the preceding claims, wherein the nucleotide sequence contains:
- an N-terminal transient signal peptide of maize cytokinin oxidase/dehydrogenase 1;
- a sequence encoding the antimicrobial peptide which is human LL-37;
- KDEL sequence,
and it is under the control of a B1 hordein promoter.

7. The method according to claim 1, wherein the nucleotide sequence is selected from the group of sequences having at least 80%, preferably at least 90%, more preferably at least 95%, identity with one of the sequences SEQ ID NO. 1, SEQ ID NO. 2, SEQ ID NO 3, SEQ ID NO 4, most preferably the nucleotide sequence is selected from SEQ ID NO. 1, SEQ ID NO. 2, SEQ ID NO. 3, SEQ ID NO. 4.

8. Expression cassette which contains a promotor, a nucleotide sequence and a terminator; **characterized in that** the nucleotide sequence contains
- a sequence encoding an N-terminal signal peptide from a plant other than barley for translocation of a peptide through endoplasmatic reticulum, selected from N-terminal transient signal peptide of maize cytokinin oxidase/dehydrogenase 1 and N-terminal signal peptide of rice chitinase 1,
- a sequence encoding at least one antimicrobial peptide selected from a group consisting of cathelicidins, defensins and magainins, and
- optionally one or more sequences selected from the group comprising a sequence KDEL encoding a C-terminal sequence for the retention of a peptide on endoplasmatic reticulum, and a sequence encoding at least one stabilization and/or purification tag selected from a histidine tag, a maltose-binding protein, a restriction site recognized by a protease, histidine tag, SUMOstar, putative barley SUMO sequence, DAMP4 biosurfactant protein and/or flexible binding sequence (GGGGS)₂;
**and in that** the promoter is a grain-specific or a constitutive promoter selected from a ubiquitin promoter, a B1 hordein promoter, a trypsin inhibitor barley promoter, an oat globulin promoter;
and wherein the terminator is preferably a Nos terminator.

9. The expression cassette according to claim 8, wherein
the nucleotide sequence contains:
- an N-terminal transient signal peptide of maize cytokinin oxidase/dehydrogenase 1;
- a sequence encoding the antimicrobial peptide which is human LL-37;
- KDEL sequence;
the promoter is a B1 hordein promoter.

10. The expression cassette according to claim 8, having the sequence having at least 80%, preferably at least 90%, more preferably at least 95%, identity with one of the sequences SEQ ID NO. 15, SEQ ID NO. 16, SEQ ID NO. 17, SEQ ID NO. 18, SEQ ID NO. 19, SEQ ID NO. 20, SEQ ID NO. 21.

11. A barley plant, **characterized in that** it contains in its genome a nucleotide sequence containing
- a sequence encoding an N-terminal signal peptide from a plant other than barley for translocation of a peptide through endoplasmatic reticulum, selected from N-terminal transient signal peptide of maize cytokinin oxidase/dehydrogenase 1 and N-terminal signal peptide of rice chitinase 1,
- a sequence encoding at least one antimicrobial peptide selected from a group consisting of cathelicidins, defensins and magainins, and
- optionally one or more sequences selected from the group comprising a sequence KDEL encoding a C-terminal sequence for the retention of a peptide on endoplasmatic reticulum, and a sequence encoding at least one stabilization and/or purification tag selected from a histidine tag, a maltose-binding protein, a restriction site recognized by a protease histidine tag, SUMOstar, putative barley SUMO sequence, DAMP4 biosurfactant protein and/or flexible binding sequence (GGGGS)₂;
under the control of a promoter which is a grain-specific or a constitutive promoter selected from a ubiquitin promoter, a B1 hordein promoter, a trypsin inhibitor barley promoter, an oat globulin promoter.

12. The barley plant according to claim 11, which contains in its genome a nucleotide sequence containing
- an N-terminal transient signal peptide of maize cytokinin oxidase/dehydrogenase 1;
- a sequence encoding the antimicrobial peptide which is human LL-37;
- KDEL sequence,
wherein the nucleotide sequence is under the control of a B1 hordein promoter or a ubiquitin promoter.

13. A method of production of recombinant antimicrobial peptides selected from a group consisting of cathelicidins, defensins and magainins, comprising the following steps:
(a) providing a barley plant comprising in its genome a nucleotide sequence containing
- a sequence encoding an N-terminal signal peptide from a plant other than barley for translocation of a peptide through endoplasmatic reticulum, selected from N-terminal transient signal peptide of maize cytokinin oxidase/dehydrogenase 1 and N-terminal signal peptide of rice chitinase 1,
- a sequence encoding at least one antimicrobial peptide selected from a group consisting of cathelicidins, defensins and magainins, and
- optionally one or more sequences selected from the group comprising a sequence KDEL encoding a C-terminal sequence for the retention of a peptide on endoplasmatic reticulum, and a sequence encoding at least one stabilization and/or purification tag selected from a histidine tag, a maltose-binding protein, a restriction site recognized by a protease histidine tag, SUMOstar, putative barley SUMO sequence, DAMP4 biosurfactant protein and/or flexible binding sequence (GGGGS)₂;
under the control of a promoter which is a grain-specific or a constitutive promoter selected from a ubiquitin promoter, a B 1 hordein promoter, a trypsin inhibitor barley promoter, an oat globulin promoter.
(b) cultivation of the barley plant until grains are formed or, optionally, until the grains are ripe;
(c) isolation of the recombinant antimicrobial peptide or a chimeric variant thereof from the barley caryopsises and/or grains.

14. The method according to claim 13, wherein in step (a), provided is a barley plant comprising in its genome a nucleotide sequence containing
- an N-terminal transient signal peptide of maize cytokinin oxidase/dehydrogenase 1;
- a sequence encoding the antimicrobial peptide which is human LL-37;
- KDEL sequence,
which is under the control of a B 1 hordein promoter.

## Patentansprüche

1. Verfahren zur Herstellung einer Gerstenpflanze, die mindestens ein antimikrobielles Peptid produziert, umfassend den Schritt der Insertion einer Nukleotidsequenz durch Transformation in das Genom der Gerstenpflanze, worin die Nukleotidsequenz enthält:
- eine Sequenz, die ein N-terminales Signalpeptid aus einer anderen Pflanze als Gerste für die Translokation eines Peptids durch das endoplasmatische Retikulum kodiert, ausgewählt aus dem N-terminalen transienten Signalpeptid der Cytokininoxidase/Dehydrogenase 1 aus Mais und dem N-terminalen Signalpeptid der Chitinase 1 aus Reis, und
- eine Sequenz, die mindestens ein antimikrobielles Peptid kodiert, ausgewählt aus einer Gruppe bestehend aus Cathelicidinen, Defensinen und Magaininen,
und wobei die Nukleotidsequenz unter der Kontrolle eines getreidespezifischen oder eines konstitutiven Promotors steht, ausgewählt aus einem Ubiquitin-Promotor, einem B1-Hordein-Promotor, einem Trypsin-Inhibitor-Gersten-Promotor und einem Hafer-Globulin-Promotor.

2. Verfahren nach Anspruch 1, **dadurch gekennzeichnet, dass** die Nukleotidsequenz weiterhin eine oder mehrere Sequenzen enthält, ausgewählt aus der Gruppe bestehend aus:
- eine Sequenz, die eine C-terminale Sequenz KDEL für die Retention eines Peptids auf dem endoplasmatischen Retikulum kodiert;
- eine Sequenz, die mindestens einen Stabilisierungs- und/oder Reinigungs-Tag kodiert, ausgewählt aus einem Histidin-Tag, einem Maltose-bindenden Protein, einer von einer Protease erkannten Restriktionsstelle, einem Histidin-Tag, SUMOstar, einer mutmaßlichen Gersten-SUMO-Sequenz, einem DAMP4-Biosurfactant-Protein und/oder flexibel Bindungssequenz (GGGGS)₂.

3. Verfahren nach Anspruch 1 oder 2, wobei das antimikrobielle Peptid LL-37 ist.

4. Verfahren nach einem der vorhergehenden Ansprüche, wobei der getreidespezifische Promotor der B1-Hordein-Promotor ist.

5. Verfahren nach Anspruch 2, wobei der Stabilisierungs- und/oder Reinigungs-Tag der Histidin-Tag und/oder die flexible Bindungssequenz (GGGGS)₂ ist.

6. Verfahren nach einem der vorhergehenden Ansprüche, wobei die Nukleotidsequenz enthält:
- ein N-terminales transientes Signalpeptid der Cytokininoxidase/Dehydrogenase 1 aus Mais;
- eine Sequenz, die das antimikrobielle Peptid kodiert, bei dem es sich um menschliches LL-37 handelt;
- KDEL-Sequenz,
und es steht unter der Kontrolle eines B 1-Hordein-Promotors.

7. Verfahren nach Anspruch 1, wobei die Nukleotidsequenz aus der Gruppe von Sequenzen ausgewählt ist, die mindestens 80 %, vorzugsweise mindestens 90 %, besonders bevorzugt mindestens 95 %, Identität mit einer der Sequenzen SEQ ID NO. 1, SEQ ID NO. 2, SEQ ID NO. 3, SEQ ID NO. 4, am meisten bevorzugt ist die Nukleotidsequenz ausgewählt aus SEQ ID NO. 1, SEQ ID NO. 2, SEQ ID NO. 3, SEQ ID NO. 4.

8. Expressionskassette, die einen Promotor, eine Nukleotidsequenz und einen Terminator enthält; **dadurch gekennzeichnet, dass** die Nukleotidsequenz enthält, worin die Nukleotidsequenz enthält:
- eine Sequenz, die ein N-terminales Signalpeptid aus einer anderen Pflanze als Gerste für die Translokation eines Peptids durch das endoplasmatische Retikulum kodiert, ausgewählt aus dem N-terminalen transienten Signalpeptid der Cytokininoxidase/Dehydrogenase 1 aus Mais und dem N-terminalen Signalpeptid der Chitinase 1 aus Reis, und
- eine Sequenz, die mindestens ein antimikrobielles Peptid kodiert, ausgewählt aus einer Gruppe bestehend aus Cathelicidinen, Defensinen und Magaininen, und
- optional eine oder mehrere Sequenzen, ausgewählt aus der Gruppe, umfassend eine Sequenz KDEL, die eine C-terminale Sequenz für die Retention eines Peptids auf dem endoplasmatischen Retikulum kodiert, und eine Sequenz, die mindestens einen Stabilisierungs- und/oder Reinigungs-Tag kodiert, ausgewählt aus einem Histidin-Tag, einem Maltose-bindendes Protein, eine von einer Protease erkannte Restriktionsstelle, Histidin-Tag, SUMOstar, mutmaßliche Gersten-SUMO-Sequenz, DAMP4-Biosurfactant-Protein und/oder flexible Bindungssequenz (GGGGS)₂;
und dass der Promotor ein getreidespezifischer oder konstitutiver Promotor ist, ausgewählt aus einem Ubiquitin-Promotor, einem B1-Hordein-Promotor, einem Trypsin-Inhibitor-Gersten-Promotor, einem Hafer-Globulin-Promotor;
und wobei der Terminator vorzugsweise ein Nos-Terminator ist.

9. Expressionskassette nach Anspruch 8, wobei
die Nukleotidsequenz enthält:
- ein N-terminales transientes Signalpeptid der Cytokininoxidase/Dehydrogenase 1 aus Mais;
- eine Sequenz, die das antimikrobielle Peptid kodiert, bei dem es sich um menschliches LL-37 handelt;
- KDEL-Sequenz;
der Promotor ist ein B1-Hordein-Promotor.

10. Expressionskassette nach Anspruch 8, wobei die Sequenz mindestens 80 %, vorzugsweise mindestens 90 %, noch bevorzugter mindestens 95 %, Identität mit einer der Sequenzen SEQ ID NO. 15, SEQ ID NO. 16, SEQ ID NO. 17, SEQ ID NO. 18, SEQ ID NO. 19, SEQ ID NO. 20, SEQ ID NO. 21.

11. Eine Gerstenpflanze, **dadurch gekennzeichnet, dass** sie in ihrem Genom eine Nukleotidsequenz enthält
- eine Sequenz, die ein N-terminales Signalpeptid aus einer anderen Pflanze als Gerste für die Translokation eines Peptids durch das endoplasmatische Retikulum kodiert, ausgewählt aus dem N-terminalen transienten Signalpeptid der Cytokininoxidase/Dehydrogenase 1 aus Mais und dem N-terminalen Signalpeptid der Chitinase 1 aus Reis ,
- eine Sequenz, die mindestens ein antimikrobielles Peptid kodiert, ausgewählt aus einer Gruppe bestehend aus Cathelicidinen, Defensinen und Magaininen, und
- optional eine oder mehrere Sequenzen, ausgewählt aus der Gruppe, umfassend eine Sequenz KDEL, die eine C-terminale Sequenz für die Retention eines Peptids auf dem endoplasmatischen Retikulum kodiert, und eine Sequenz, die mindestens einen Stabilisierungs- und/oder Reinigungs-Tag kodiert, ausgewählt aus einem Histidin-Tag, einem Maltose-bindendes Protein, eine Restriktionsstelle, die von einem Protease-Histidin-Tag erkannt wird, SUMOstar, mutmaßliche Gersten-SUMO-Sequenz, DAMP4-Biosurfactant-Protein und/oder flexible Bindungssequenz (GGGGS)₂;
unter der Kontrolle eines Promotors, der ein getreidespezifischer oder konstitutiver Promotor ist, ausgewählt aus einem Ubiquitin-Promotor, einem B1-Hordein-Promotor, einem Trypsin-Inhibitor-Gersten-Promotor und einem Hafer-Globulin-Promotor.

12. Gerstenpflanze nach Anspruch 11, die in ihrem Genom eine Nukleotidsequenz enthält, worin die Nukleotidsequenz enthält
- ein N-terminales transientes Signalpeptid der Cytokininoxidase/Dehydrogenase 1 aus Mais;
- eine Sequenz, die das antimikrobielle Peptid kodiert, bei dem es sich um menschliches LL-37 handelt;
- KDEL-Sequenz,
wobei die Nukleotidsequenz unter der Kontrolle eines B1-Hordein-Promotors oder eines Ubiquitin-Promotors steht.

13. Verfahren zur Herstellung rekombinanter antimikrobieller Peptide, ausgewählt aus einer Gruppe bestehend aus Cathelicidinen, Defensinen und Magaininen, umfassend die folgenden Schritte:
(a) Bereitstellen einer Gerstenpflanze, die in ihrem Genom eine Nukleotidsequenz enthält, worin die Nukleotidsequenz enthält:
- eine Sequenz, die ein N-terminales Signalpeptid aus einer anderen Pflanze als Gerste für die Translokation eines Peptids durch das endoplasmatische Retikulum kodiert, ausgewählt aus dem N-terminalen transienten Signalpeptid der Cytokininoxidase/Dehydrogenase 1 aus Mais und dem N-terminalen Signalpeptid der Chitinase 1 aus Reis,
- eine Sequenz, die mindestens ein antimikrobielles Peptid kodiert, ausgewählt aus einer Gruppe bestehend aus Cathelicidinen, Defensinen und Magaininen, und
- optional eine oder mehrere Sequenzen, ausgewählt aus der Gruppe, umfassend eine Sequenz KDEL, die eine C-terminale Sequenz für die Retention eines Peptids auf dem endoplasmatischen Retikulum kodiert, und eine Sequenz, die mindestens einen Stabilisierungs- und/oder Reinigungs-Tag kodiert, ausgewählt aus einem Histidin-Tag, einem Maltose-bindendes Protein, eine Restriktionsstelle, die von einem Protease-Histidin-Tag erkannt wird, SUMOstar, mutmaßliche Gersten-SUMO-Sequenz, DAMP4-Biosurfactant-Protein und/oder flexible Bindungssequenz (GGGGS)₂;
unter der Kontrolle eines Promotors, der ein getreidespezifischer oder konstitutiver Promotor ist, ausgewählt aus einem Ubiquitin-Promotor, einem B 1-Hordein-Promotor, einem Trypsin-Inhibitor-Gersten-Promotor und einem Hafer-Globulin-Promotor.
(b) Kultivierung der Gerstenpflanze bis zur Bildung von Körnern oder optional bis zur Reife der Körner;
(c) Isolierung des rekombinanten antimikrobiellen Peptids oder einer chimären Variante davon aus den Gerstenkaryopsen und/oder -körnern.

14. Verfahren nach Anspruch 13, wobei in Schritt (a) eine Gerstenpflanze bereitgestellt wird, die in ihrem Genom eine Nukleotidsequenz enthält, die Nukleotidsequenz enthaltend
- ein N-terminales transientes Signalpeptid der Cytokininoxidase/Dehydrogenase 1 aus Mais;
- eine Sequenz, die das antimikrobielle Peptid kodiert, bei dem es sich um menschliches LL-37 handelt;
- KDEL-Sequenz,
welches unter der Kontrolle eines B1-Hordein-Promotors steht.

## Revendications

1. Procédé de production d'un plant d'orge produisant au moins un peptide antimicrobien, comprenant l'étape d'insertion par transformation d'une séquence nucléotidique contenant
- une séquence codant pour un peptide signal N-terminal provenant d'une plante autre que l'orge pour la translocation d'un peptide à travers le réticulum endoplasmatique, sélectionnée parmi le peptide signal transitoire N-terminal de la cytokinine oxydase/déshydrogénase 1 du maïs et le peptide signal N-terminal de la chitinase 1 du riz , et
- une séquence codant pour au moins un peptide antimicrobien choisi dans un groupe constitué des cathélicidines, des défensines et des magainines,
dans le génome de la plante d'orge ;
et dans lequel la séquence nucléotidique est sous le contrôle d'un promoteur spécifique aux céréales ou d'un promoteur constitutif choisi parmi un promoteur d'ubiquitine, un promoteur d'hordéine B1, un promoteur d'orge inhibiteur de trypsine, un promoteur de globuline d'avoine.

2. Procédé selon la revendication 1, **caractérisé en ce que** la séquence nucléotidique contient en outre une ou plusieurs séquences choisies dans le groupe constitué de
- une séquence codant pour une séquence C-terminale KDEL pour la rétention d'un peptide sur le réticulum endoplasmatique ;
- une séquence codant pour au moins un marqueur de stabilisation et/ou de purification choisi parmi un marqueur histidine, une protéine liant le maltose, un site de restriction reconnu par une protéase, un marqueur histidine, SUMOstar, une séquence putative SUMO d'orge, une protéine biosurfactante DAMP4 et/ou flexible séquence de liaison (GGGGS)₂.

3. Procédé selon la revendication 1 ou 2, dans lequel le peptide antimicrobien est le LL-37.

4. Procédé selon l'une quelconque des revendications précédentes, dans lequel le promoteur spécifique des grains est le promoteur de l'hordéine B1.

5. Procédé selon la revendication 2, dans lequel le marqueur de stabilisation et/ou de purification est le marqueur histidine et/ou la séquence de liaison flexible (GGGGS)₂.

6. Procédé selon l'une quelconque des revendications précédentes, dans lequel la séquence nucléotidique contient:
- un peptide signal transitoire N-terminal de la cytokinine oxydase/déshydrogénase 1 du maïs ;
- une séquence codant pour le peptide antimicrobien qui est le LL-37 humain ;
- séquence KDEL,
et il est sous le contrôle d'un promoteur de l'hordéine B1.

7. Procédé selon la revendication 1, dans lequel la séquence nucléotidique est choisie dans le groupe des séquences présentant au moins 80%, de préférence au moins 90%, plus préférentiellement au moins 95% d'identité avec l'une des séquences SEQ ID NO. 1, SEQ ID NO. 2, SEQ ID NO. 3, SEQ ID NO. 4, de préférence la séquence nucléotidique est choisie parmi SEQ ID NO. 1, SEQ ID NO. 2, SEQ ID NO. 3, SEQ ID NO. 4.

8. Cassette d'expression qui contient un promoteur, une séquence nucléotidique et un terminateur; **caractérisé en ce que** la séquence nucléotidique contient
- une séquence codant pour un peptide signal N-terminal provenant d'une plante autre que l'orge pour la translocation d'un peptide à travers le réticulum endoplasmatique, sélectionnée parmi le peptide signal transitoire N-terminal de la cytokinine oxydase/déshydrogénase 1 du maïs et le peptide signal N-terminal de la chitinase 1 du riz,
- une séquence codant pour au moins un peptide antimicrobien choisi dans un groupe constitué des cathélicidines, des défensines et des magainines, et
- éventuellement une ou plusieurs séquences sélectionnées dans le groupe comprenant une séquence KDEL codant pour une séquence C-terminale de rétention d'un peptide sur le réticulum endoplasmatique, et une séquence codant pour au moins un marqueur de stabilisation et/ou de purification choisi parmi un marqueur histidine, un protéine de liaison au maltose, un site de restriction reconnu par une protéase, un marqueur histidine, SUMOstar, une séquence putative SUMO d'orge, une protéine biosurfactante DAMP4 et/ou une séquence de liaison flexible (GGGGS)₂;
**et en ce que** le promoteur est un promoteur spécifique aux céréales ou un promoteur constitutif choisi parmi un promoteur d'ubiquitine, un promoteur d'hordéine B1, un promoteur d'orge inhibiteur de trypsine, un promoteur de globuline d'avoine ;
et dans lequel le terminateur est de préférence un terminateur Nos.

9. Cassette d'expression selon la revendication 8, dans laquelle
la séquence nucléotidique contient:
- un peptide signal transitoire N-terminal de la cytokinine oxydase/déshydrogénase 1 du maïs;
- une séquence codant pour le peptide antimicrobien qui est le LL-37 humain;
- séquence KDEL;
le promoteur est un promoteur de l'hordéine B1.

10. Cassette d'expression selon la revendication 8, dont la séquence présente au moins 80%, de préférence au moins 90%, plus préférablement au moins 95% d'identité avec l'une des séquences SEQ ID NO. 15, SEQ ID NO. 16, SEQ ID NO. 17, SEQ ID NO. 18, SEQ ID NO. 19, SEQ ID NO. 20, SEQ ID NO. 21.

11. Plante d'orge, **caractérisée en ce qu'**elle contient dans son génome une séquence nucléotidique contenant
- une séquence codant pour un peptide signal N-terminal provenant d'une plante autre que l'orge pour la translocation d'un peptide à travers le réticulum endoplasmatique, sélectionnée parmi le peptide signal transitoire N-terminal de la cytokinine oxydase/déshydrogénase 1 du maïs et le peptide signal N-terminal de la chitinase 1 du riz ,
- une séquence codant pour au moins un peptide antimicrobien choisi dans un groupe constitué des cathélicidines, des défensines et des magainines, et
- éventuellement une ou plusieurs séquences sélectionnées dans le groupe comprenant une séquence KDEL codant pour une séquence C-terminale de rétention d'un peptide sur le réticulum endoplasmatique, et une séquence codant pour au moins un marqueur de stabilisation et/ou de purification choisi parmi un marqueur histidine, un protéine de liaison au maltose, un site de restriction reconnu par une protéase, un marqueur histidine, SUMOstar, séquence SUMO putative de l'orge, protéine biosurfactante DAMP4 et/ou séquence de liaison flexible (GGGGS)₂;
sous le contrôle d'un promoteur qui est un promoteur spécifique aux céréales ou un promoteur constitutif choisi parmi un promoteur d'ubiquitine, un promoteur d'hordéine B1, un promoteur d'orge inhibiteur de trypsine, un promoteur de globuline d'avoine.

12. Plante d'orge selon la revendication 11, qui contient dans son génome une séquence nucléotidique contenant
- un peptide signal transitoire N-terminal de la cytokinine oxydase/déshydrogénase 1 du maïs ;
- une séquence codant pour le peptide antimicrobien qui est le LL-37 humain ;
- séquence KDEL,
la séquence nucléotidique étant sous le contrôle d'un promoteur de l'hordéine B1 ou d'un promoteur de l'ubiquitine.

13. Procédé de production de peptides antimicrobiens recombinants choisis dans un groupe constitué des cathélicidines, défensines et magainines, comprenant les étapes suivantes:
(a) fournir un plant d'orge comprenant dans son génome une séquence nucléotidique contenant
- une séquence codant pour un peptide signal N-terminal provenant d'une plante autre que l'orge pour la translocation d'un peptide à travers le réticulum endoplasmatique, sélectionnée parmi le peptide signal transitoire N-terminal de la cytokinine oxydase/déshydrogénase 1 du maïs et le peptide signal N-terminal de la chitinase 1 du riz,
- une séquence codant pour au moins un peptide antimicrobien choisi dans un groupe constitué des cathélicidines, des défensines et des magainines, et
- éventuellement une ou plusieurs séquences sélectionnées dans le groupe comprenant une séquence KDEL codant pour une séquence C-terminale de rétention d'un peptide sur le réticulum endoplasmatique, et une séquence codant pour au moins un marqueur de stabilisation et/ou de purification choisi parmi un marqueur histidine, un protéine de liaison au maltose, un site de restriction reconnu par une protéase, un marqueur histidine, SUMOstar, séquence SUMO putative de l'orge, protéine biosurfactante DAMP4 et/ou séquence de liaison flexible (GGGGS)₂;
sous le contrôle d'un promoteur qui est un promoteur spécifique aux céréales ou un promoteur constitutif choisi parmi un promoteur d'ubiquitine, un promoteur d'hordéine B1, un promoteur d'orge inhibiteur de trypsine, un promoteur de globuline d'avoine;
(b) culture du plant d'orge jusqu'à la formation des grains ou, éventuellement, jusqu'à ce que les grains soient mûrs ;
(c) l'isolement du peptide antimicrobien recombinant ou d'un variant chimérique de celui-ci à partir des caryopses et/ou des grains d'orge.

14. Procédé selon la revendication 13, dans lequel à l'étape (a), il est fourni un plant d'orge comprenant dans son génome une séquence nucléotidique contenant
- un peptide signal transitoire N-terminal de la cytokinine oxydase/déshydrogénase 1 du maïs ;
- une séquence codant pour le peptide antimicrobien qui est le LL-37 humain ;
- séquence KDEL,
qui est sous le contrôle d'un promoteur de l'hordéine B1.
